(19) Europäisches Patentamt
European Patent Office
Office européen des brevets

(11) **EP 2 226 637 A1**

(12) # DEMANDE DE BREVET EUROPEEN

(43) Date de publication:
**08.09.2010 Bulletin 2010/36**

(51) Int Cl.:
***G01N 33/68*** *(2006.01)*

(21) Numéro de dépôt: **09305200.9**

(22) Date de dépôt: **04.03.2009**

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(71) Demandeur: **Centre National de la Recherche Scientifique (CNRS)**
**75016 Paris (FR)**

(72) Inventeurs:
- **Martinez, Jean**
  **34720, CAUX (FR)**
- **Subra, Gilles**
  **34980, SAINT GELY DU FESC (FR)**
- **Goubet, Christine**
  **34270, LES MATELLES (FR)**
- **Paramelle, David**
  **81160, SAINT JUERY (FR)**
- **Forest, Eric**
  **38120, SAINT-EGREVE (FR)**
- **Heymann, Michaël**
  **68110, ILLZACH (FR)**
- **Geourjon, Christophe**
  **69007, LYON (FR)**

(74) Mandataire: **Ahner, Francis et al**
**Cabinet Régimbeau**
**20, rue de Chazelles**
**75847 Paris Cedex 17 (FR)**

(54) **Agents de reticulation sur support solide**

(57) L'invention concerne un agent de réticulation des protéines sur support solide de formule (I)

M-L-B-A (I)

dans laquelle M est un support solide choisi dans le groupe constitué des polymères naturels et dérivés, des matrices inorganiques et des polymères de synthèse fonctionnalisés, L est un linker, la liaison entre L et B est clivable, B est une chaîne linéaire hydrocarbonée en $C_1$ à $C_{60}$, de préférence $C_5$ à $C_{10}$, présentant éventuellement une ou plusieurs double ou triple liaisons dont un ou plusieurs carbones peuvent être remplacés par O, NH, -C=O,

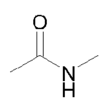

ou

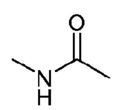

et un ou plusieurs autres carbones peuvent être substitués par un groupe $C_{1-10}$ alkyle, $C_{2-10}$ alcényle, $C_{2-10}$ alcynyle, aryle ou aryl($C_{1-10}$ alkyle), et B forme avec L et A une liaison amide, et A est un agent de réticulation des protéines présentant la formule (II) ou (IIbis) ou (III) ou (IIIbis)

EP 2 226 637 A1

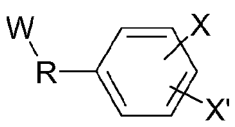 (II) 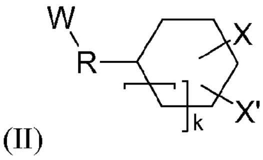 (IIbis)

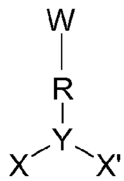 (III) -W-R-X (IIIbis)

dans lesquelles
W est NH ou C=O,
Y est N ou CH,
R est absent ou représente un groupe $C_{1-10}$ alkyle, $C_{2-10}$ alcényle ou $C_{2-10}$ alcynyle,
k est 0, 1, 2 ou 3,
X et X', identiques ou différents, sont une fonction réactive des protéines.

## Description

L'invention concerne le domaine des analyses structurales des protéines par spectrométrie de masse.

L'analyse des structures tridimensionnelles des protéines et de leurs interactions avec leur environnement (*e.g.* ADN, protéines, membranes) est très importante pour la compréhension des fonctions biologiques des protéines dans leur milieu naturel.

Afin d'obtenir ces données structurales à haute résolution, la RMN et la DRX sont des méthodes d'analyse couramment utilisées. Cependant, ces méthodes sont très limitées par certaines contraintes : cristallisation de protéine, utilisation de grande quantité de matériel (5 à 10 mg de protéines).

L'utilisation d'agents de réticulation chimique conjointement à la spectrométrie de masse est une méthode de plus en plus fréquente pour la détermination de données structurales à basse résolution permettant de s'affranchir des contraintes précédentes.

Cette approche consiste à effectuer une digestion enzymatique des protéines du milieu réactionnel après avoir réalisé la réaction de réticulation en solution. Le mélange de peptides obtenu est alors analysé par spectrométrie de masse afin d'identifier les peptides modifiés par les agents de réticulation. Des données structurales sont alors obtenues de cette identification. Cette stratégie a été utilisée dans le cadre d'études structurales à basse résolution de protéines ou d'interactions protéine-protéine.

Les agents de réticulation chimique possèdent une ou deux fonctions réactives reliées par un bras espaceur. Ces fonctions réactives sont capables de réagir avec les chaînes latérales des aminoacides de protéines. Après identification des résidus aminoacides des protéines modifiés par les deux fonctions réactives, des contraintes de distances au sein de ces protéines peuvent être déterminées grâce à la longueur connue du bras espaceur de l'agent de réticulation chimique.

Des agents homobifonctionnels, tels que les esters bis-imido ou les halogénures dialkyles furent mis au point et utilisés sur des protéines dès les années 1950.

Toutefois, l'utilisation d'agents de réticulation des protéines en solution pose un problème majeur de détection de peptides d'intérêts dans un milieu mixte complexe comprenant à la fois les peptides modifiés et non modifiés par les agents de réticulation.

Il existe donc un besoin important en une méthode capable d'enrichir le milieu qui va être analysé en spectrométrie de masse en les peptides modifiés par les agents de réticulation voire d'isoler de manière spécifique les peptides réticulés et ainsi faciliter les analyses par spectrométrie de masse.

Afin de résoudre ce problème, le Demandeur a mis au point de façon inattendue une méthode d'extraction spécifique de peptides réticulés pour l'étude de structure tridimensionnelle basse résolution de protéines. Cette méthode comprend le développement d'agents de réticulation chimique des protéines sur support solide.

En particulier, le Demandeur a montré qu'il est possible à la fois de synthétiser un agent de réticulation chimique des protéines directement sur un support solide tout en conservant l'accessibilité de la protéine à l'agent de réticulation fixé au support solide et de l'enzyme à la protéine fixée sur l'agent de réticulation.

Résumé de l'invention

Le premier objet de l'invention concerne un agent de réticulation des protéines sur support solide de formule (I)

M-L-B-A (I)

dans laquelle
M est un support solide choisi dans le groupe constitué des polymères naturels et dérivés, des matrices inorganiques et des polymères de synthèse fonctionnalisés,
L est un linker,
la liaison entre L et B est clivable,
B est une chaîne linéaire hydrocarbonée en $C_1$ à $C_{60}$, de préférence $C_5$ à $C_{10}$, présentant éventuellement une ou plusieurs double ou triple liaisons dont un ou plusieurs carbones peuvent être remplacés par O, NH, -C=O,

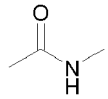

ou

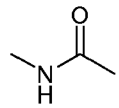

et un ou plusieurs autres carbones peuvent être substitués par un groupe $C_{1-10}$ alkyle, $C_{2-10}$ alcényle, $C_{2-10}$ alcynyle, aryle ou aryl($C_{1-10}$ alkyle), et
B forme avec L et A une liaison amide, et
A est un agent de réticulation des protéines présentant la formule (II) ou (IIbis) ou (III) ou (IIIbis)

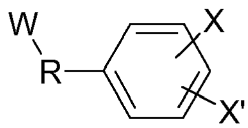 (II) 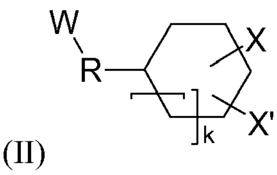 (IIbis)

(III) 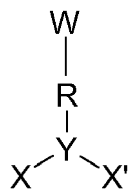

-W-R-X (IIIbis)

dans lesquelles
W est NH ou C=O,
Y est N ou CH,
R est absent ou est un groupe $C_{1-10}$ alkyle, $C_{2-10}$ alcényle ou $C_{2-10}$ alcynyle,
k est 0, 1, 2 ou 3,
X et X', identiques ou différents, sont une fonction réactive des protéines.

Un autre objet de l'invention concerne un procédé de préparation d'un agent de réticulation selon l'invention, comprenant les étapes suivantes :

- éventuellement couplage peptidique entre la fonction amine du support et la fonction acide carboxylique d'un acide aminé dont la fonction amine est protégée, puis déprotection de la fonction amine,
- couplage peptidique entre un diacide ou un anhydride et la fonction amine du support ou du composé obtenu à l'étape précédente, et
- éventuellement couplage peptidique entre la fonction acide du composé obtenu à l'étape précédente et la fonction amine d'un acide aminé dont la fonction acide est protégée, puis déprotection de la fonction acide.

Un autre objet de l'invention concerne l'utilisation d'un agent de réticulation sur support solide selon l'invention pour isoler les peptides réticulés avant leur analyse en spectrométrie de masse.

Un autre objet de l'invention concerne une méthode d'analyse structurale d'une protéine ou d'un complexe de protéines comprenant les étapes suivantes :

a) réticulation de la protéine ou du complexe protéique sur l'agent de réticulation sur support solide selon l'une quelconque des revendications 1 à 8 par les fonctions X et/ou X',
b) lavage(s) pour éliminer les protéines ou complexes de protéines non réticulés,
c) digestion enzymatique de la protéine ou complexe de protéines fixé à l'agent de réticulation sur support solide selon l'une quelconque des revendications 1 à 8,
d) lavage(s) pour éliminer les peptides non réticulés,
e) clivage du support solide entre L et B, et

f) analyse par spectrométrie de masse.

Description détaillée

DEFINITIONS

Par groupement « $C_{1-10}$ alkyle », on entend une chaîne hydrocarbonée saturée, linéaire ou ramifiée, comportant de 1 à 10 atomes de carbone, comme par exemple un groupement, méthyle, éthyle, isopropyle, *tertio*-butyle, pentyle, etc.

Par groupement « $C_{2-10}$ alcényle », on entend une chaîne hydrocarbonée, linéaire ou ramifiée, comportant au moins une insaturation et comportant de 2 à 10 atomes de carbone, comme par exemple un groupement éthényle, propényle, 2,4-hexadiényle, etc.

Par groupement « $C_{2-10}$ alcynyle », on entend une chaîne hydrocarbonée, linéaire ou ramifiée, comportant au moins une triple liaison et comportant de 2 à 10 atomes de carbone, comme par exemple un groupement éthynyle, propynyle, etc.

Par groupement « aryle », on entend un groupement aromatique, comportant de préférence de 5 à 10 atomes de carbone, comprenant un ou plusieurs cycles et comprenant éventuellement un hétéroatome, en particulier un oxygène, un azote ou un soufre, comme par exemple un groupement phényle, furane, indole, pyridine, naphtalène, etc.

Le terme « halogène » désigne un fluor, un brome, un chlore ou un iode.

LES LINKERS

Avantageusement, le linker L de l'agent de réticulation de l'invention est choisi dans le groupe constitué des linkers chlorotrityl, 2-chloro chlorotrityl, 4-hydroxyméthyl benzamido, 4-hydroxyméthyl phénoxy, 3-méthoxy 4-hydroxyphénylphénoxy , 4-Hydroxyméthyl-phénylacétamido, hydroxyméthyl-3,5-diméthoxyphénoxyalkyle, Rink signifiant 4-[2',4'-diméthoxyphényl-(9-fluorométhyloxycarbonyl)-aminométhyl]phénoxy-), Sieber amide (Sieber signifiant 9-amino-xanthèn-3-yloxy-), aminométhyl-3,5-diméthoxy-phénoxyalkyle, aminométhyl-3,5-diméthoxyphénoxyalkyle, 4-méthylbenzhydrylamine, hydroxy(2-chlorophényl)méthyl, 3 nitro 4 hydroxyméthyl benzamido et linker pipecolique (EP08305512.9).

De préférence, le linker L est un linker acidolabile et de préférence, de type Rink Amide, par exemple le linker Fmoc-Rink Amide. Le groupement Fmoc protège l'amine du linker. Si la combinaison M-L n'est pas commercialement accessible, il est possible de greffer le linker sur la matrice. Dans le cas du linker Fmoc-Rink Amide, il peut être greffé sur la matrice à l'aide d'une réaction de couplage entre une amine primaire du de la matrice et l'acide Fmoc-Rink Amide-OH activé dans une solution de diisopropylcarbodiimide (DIC) et de 1-hydroxybenzotriazole (HOBt) dans du DMF. Le nombre de mole de fonctions Rink Amide (RA) par gramme de résine, appelé charge de la résine, peut être évalué à l'aide d'un dosage du dibenzofulvène libéré lors de la déprotection de la fonction amine du linker Rink Amide en présence de pipéridine.

LES BRAS ESPACEURS

L'utilité des bras espaceurs est d'améliorer l'accessibilité des fonctions réactives en les éloignant de la matrice.

De préférence, le bras espaceur B présente la formule (IV)

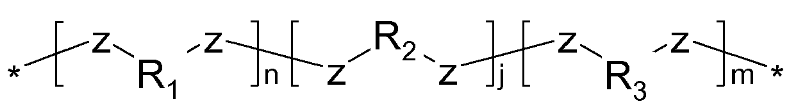

(IV)

dans laquelle Z est NH ou C=O,
n et m sont 1 ou 0,
j est un entier compris entre 1 et 60

$R_1$, $R_2$ et $R_3$, identiques ou différents, sont une chaîne hydrocarbonée en $C_1$ à $C_6$ dont un ou plusieurs carbones peuvent être remplacés par O et un ou plusieurs autres carbones peuvent être substitués par un groupe alkyle, aryle ou arylalkyle.

Avantageusement, B présente l'une des formules (V) à (IX)

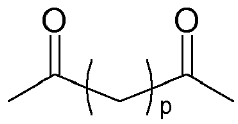

(V)

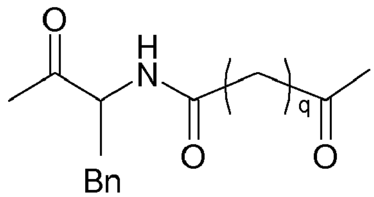

(VI)

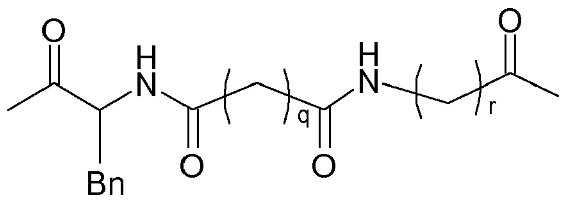

(VII)

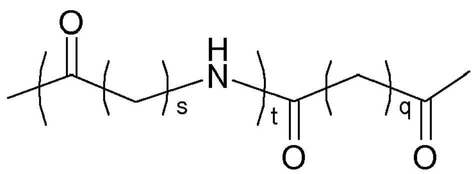

(VIII)

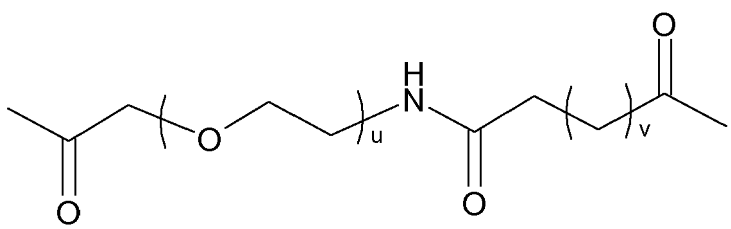

(IX)

dans lesquelles p, q, r, s, u et v sont des entiers de 1 à 5 et t est 1 ou 0.

LES MATRICES

Avantageusement, le support solide de l'agent de réticulation de l'invention est choisi dans le groupe constitué des polymères naturels et dérivés, des matrices inorganiques, des polymères de synthèse.

Les polymères naturels et dérivés appropriés comprennent les polysaccharides et leurs dérivés.

Les matrices inorganiques appropriées comprennent les supports solides dérivés de la silice, comme la silice fonctionnalisée commercialisée sous le nom silicycle® ou les particules d'or. Les polymères de synthèse appropriés comprennent les polymères de synthèse microporeux, macroporeux ou composites.

Les polymères de synthèse microporeux appropriés comprennent les polymères de styrène, les copolymères de *N,N*-diméthylacrylamide et de *N,N*'-bis-acryloyoléthylène diamine, les copolymères d'acrylamide et de bis/mono-acrylamido-PEG.

Avantageusement encore, le support solide de l'agent de réticulation de l'invention est choisi dans le groupe constitué de Sepharose®, Synbeads®, PEGA, ChemMatrix®.

De manière particulièrement préférée, le support solide de l'agent de réticulation de l'invention est la résine

Synbeads®.

❖ **Les polymères naturels et dérivés**

Les matrices composées de polymères naturels sont généralement formées à partir de polysaccharides tels que de l'agarose, cellulose ou autres dérivés. Ces matériaux contiennent des fonctions hydroxyles permettant leur fonctionnalisation avec divers bras espaceurs et fonctions pouvant réagir avec des protéines. Ils sont très hydrophiles ce qui permet leur utilisation dans des milieux physiologiques. De même, ils sont relativement stables aux variations de pH. Cependant, leur utilisation comme support solide en chimie organique n'a pas été mise en évidence du fait de leur instabilité dans les solvants organiques.

Un exemple est la Sepharose®. Cette matrice est un dérivé de gel d'agarose résultant d'un enchaînement linéaire de D-galactose et de 3,6-anhydro-D-galactose.

❖ **Les matrices inorganiques**

Les matrices inorganiques sont des matériaux de structure rigide et généralement d'origine naturelle comme la silice.

Les supports solides dérivés de silice se présentent sous forme de particules rigides de différentes tailles dont la porosité est contrôlée (de 2 à 10 nm). Leur surface accessible au solvant est comprise entre 200 et 800 $m^2.g^{-1}$. Une de leur propriété essentielle est que la matrice ne gonfle dans aucun solvant et garde donc sa taille de départ.

❖ **Les polymères de synthèse**

De nombreux polymères de synthèse, utilisés couramment pour la synthèse organique sur support solide, ont été adaptés pour immobiliser des enzymes ou pour effectuer des réactions sur des substrats immobilisés.

Ces supports solides peuvent être classés en trois groupes principaux : microporeux, macroporeux et composites. La préparation de ces différents types de matrice par polymérisation d'agents de maille et d'agents de réticulation est différente et conduit à des propriétés physiques différentes.

Les polymères microporeux sont obtenus par polymérisation homogène en suspension avec un agent de maille (éventuellement fonctionnalisé) et un agent de réticulation (environ 1 %).

Par exemple, la matrice polystyrène (ou PS) utilisée en synthèse sur support solide est un polymère microporeux obtenu par polymérisation de styrène (agent de maille) et réticulé à 1 % environ avec du divinylbenzène (DVB, agent de réticulation).

Un autre exemple de résine microporeuse est la résine de Sheppard de type polyamide. Cette matrice est synthétisée par copolymérisation de *N,N*-diméthylacrylamide (agent de maille) et de *N,N'*-bis-acryloyoléthylène diamine (agent de réticulation.

Afin d'augmenter les capacités de gonflement et de compatibilité de résines dans l'eau tout en gardant une bonne stabilité dans les solvants organiques, une nouvelle génération de matrice microporeuse a été développée par Meldal : la PEGA. Elle est synthétisée grâce une réaction de copolymérisation radicalaire avec de l'acrylamide et du mono acrylamido-PEG utilisés comme agents de maille et du bis-acrylamido-PEG (agent de réticulation).

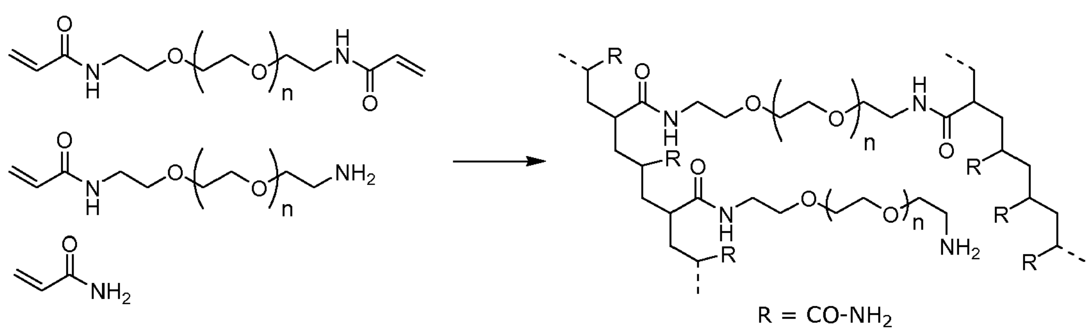

Préparation de la résine PEGA

Des résines PEGA ont été synthétisées avec des chaînes PEG de différents poids moléculaires (*e.g.* 900, 1900) afin de faire varier les capacités de gonflement de la résine pour optimiser l'accessibilité des pores à des enzymes.

La résine ChemMatri® est une matrice microporeuse hautement réticulée et entièrement composée de monomères PEG de formule suivante :

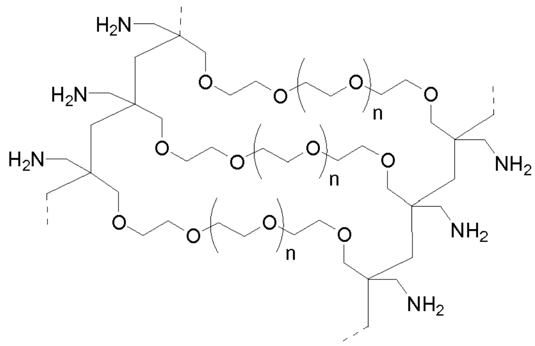

Résine ChemMatrix®

Les polymères macroporeux sont préparés de la même façon que les polymères microporeux. Cependant, un agent porogène est rajouté dans le milieu réactionnel et le taux d'agent de réticulation atteint 12% de façon à favoriser la séparation des phases.

Les billes de résine macroporeuse sont opaques, rugueuses, et ne gonflent pas ou peu quel que soit le solvant utilisé. Par conséquent, leur surface accessible est élevée et ne varie pas (entre 100 et 1000 $m^2/g$). Leur taux de réticulation élevé (supérieur à 12%) leur permet de résister à la pression. D'autre part, ces résines permettent une bonne diffusion du solvant ce qui facilite leur lavage. Ces matrices possèdent deux phases distinctes : phase microporeuse ou gélatineuse et phase macroporeuse. La phase macroporeuse permet de laisser entrer des molécules de hauts poids moléculaires comme des protéines.

La résine Synbeads®, de type méthacrylique, est une matrice macroporeuse (FR1295537). Comme toutes les matrices macroporeuses, ce support possède une structure très rigide et mécaniquement stable du à un fort taux de réticulation, ce qui permet de la manipuler facilement. Elle ne gonfle pas mais la nature du polymère méthacrylique, plus hydrophile que le polystyrène, lui assure une bonne compatibilité avec les tampons aqueux. Cette résine macroporeuse, possède des pores de diamètre d'environ 380 Å.

Les polymères de type composite sont des polymères réticulés entre 1 et 2% comme les polymères gélatineux. Ils sont le plus souvent composés de polystyrène greffé par des chaînes hydrophiles (le plus souvent de type PEG). Ces matrices possèdent donc des propriétés mixtes.

Les premiers exemples de matrices composites sont les matrices de type PEG-PS. Elles contiennent un noyau de polystyrène réticulé greffé par des chaînes polyéthylène glycol. Deux formes de ces supports solides sont commercialisées : Tentagel® et Argogel®.

Un autre exemple de résine de type PEG-PS est la résine NovaGel®.

Les Lanternes sont un autre exemple de support solide particulier de type pelliculaire s'apparentant aux supports de type composite. Elles se présentent sous une forme de petits objets (environ 8 mm de longueur) de polyéthylène inerte greffé en surface par du polyamide (*e.g.* SynPhase-PA®) ou du polystyrène (*e.g.* SynPhase-PS®) réticulé comparable à une matrice microporeuse.

LES AGENTS DE RETICULATION OU « CROSSLINKERS »

A est un agent de réticulation des protéines présentant la formule (II) ou (IIbis) ou (III) ou (IIIbis)

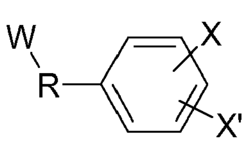 (II) 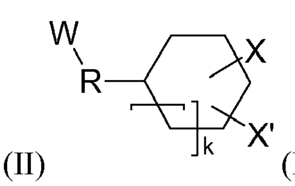 (IIbis)

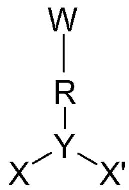

(III)

-W-R-X (IIIbis)

dans lesquelles
W est NH ou C=O,
Y est N ou CH,
R est absent ou représente un groupe $C_{1-10}$ alkyle, $C_{2-10}$ alcényle ou $C_{2-10}$ alcynyle,
k est 0 ou 1 ou 2 ou 3,
X et X', identiques ou différents, sont une fonction réactive des protéines.
W est choisi de sorte à former une liaison amide avec le bras espaceur
De préférence, Y est N.
De préférence, R est un groupe $C_{1-10}$ alkyle.
De préférence, k est 1.

Avantageusement, X et X', identiques ou différents, sont choisis dans le groupe constitué des bras incluant des fonctions imidoester, ester N-hydroxysuccinimide, N-sulfohydroxysuccinimide, ester trichlorophénol, ester pentafluorophénol, ester nitrophénol, ester N-hydroxybenzotriazole, isocyanate, isothiocyanate, N-maléimide, disulfure, 1,2-dicarbonyle, benzophénone, arylazide, amine et sulfhydril. L'agent de réticulation A comprend une ou deux fonctions réactives vis-à-vis des protéines. Il est possible de concevoir une variété très importante d'agents de réticulation A suivant la nature des fonctions réactives utilisées qui peuvent être identiques ou pas (*i.e.* agent homo ou hétérobifonctionnel).

On appelle « réticulation de la protéine ou du complexe protéique sur l'agent de réticulation sur support solide de l'invention » la réaction d'une ou plusieurs fonctions réactives X et/ou X' de l'agent de réticulation A avec un ou plusieurs groupements de la protéine ou du complexe protéique résultant en la fixation covalente de ladite protéine ou dudit complexe protéique à l'agent de réticulation A.

Les agents de réticulation homobifonctionnels possèdent deux fonctions réactives identiques pouvant réagir avec le même type de fonction.

Lors d'expériences de réticulation sur des protéines, une des deux fonctions réactives réagit sur une chaîne latérale d'un résidu aminoacide. La seconde fonction réactive réagit ensuite soit de façon intramoléculaire sur une autre chaîne latérale avoisinante, soit sur une chaîne latérale d'un résidu aminoacide appartenant à une autre protéine. Etant donné que les deux fonctions réactives sont identiques, le protocole de réaction se fait en une seule étape.

Les agents de réticulation hétérobifonctionnels possèdent deux fonctions réactives ciblant différents aminoacides.

Ils sont généralement utilisés pour réticuler des protéines en deux étapes (*e.g.* agents de réticulation possédant une fonction réactive non-spécifique et spécifique). Une fois la première fixation effectuée, il est donc possible d'effectuer une purification des protéines modifiées avant d'effectuer la réaction de la deuxième fonction réactive. Ceci peut favoriser les réactions intramoléculaires et peut permettre d'obtenir des données plus diversifiées qu'avec des agents de réticulation homobifonctionnels.

L'agent de réticulation A est immobilisé sur un support solide muni d'un linker M-L via un bras espaceur B. La liaison entre L et B est clivable, par exemple par un traitement chimique ou photochimique adéquat. Le peptide réticulé et fixé sur le support solide est ainsi libéré en solution.

LES FONCTIONS REACTIVES

X et X' sont des fonctions réactives vis-à-vis des protéines capables de réagir avec un groupement réactif des protéines.

Avantageusement, X et X', identiques ou différents, sont choisies dans le groupe constitué des fonctions réactives spécifiques des amines, des fonctions réactives spécifiques des acides carboxyliques, des fonctions réactives spécifiques des thiols, des fonctions réactives spécifiques des guanidines, des fonctions réactives non spécifiques.

**Les fonctions réactives spécifiques des amines** réagissent avec les amines primaires présentes sur les

protéines. Il existe deux types d'amine primaire au sein d'une protéine : N-terminale et N-ε des lysines. Des études ont montré qu'il est possible de les cibler de façon indépendante grâce à leur différence de valeur de pKa respectif ($pKa_{N\text{-terminale}}$ = 8 et $pKa_{N\text{-}\varepsilon\text{ des lysines}}$ = 10,5).

Trois types de fonctions sont couramment utilisées dans ce but : les imidoesters, les esters *N*-hydroxysuccinimide, les isocyanates (et isothiocyanates). Ce sont tous des agents activés électrophiles possédant un bon groupe partant par substitution nucléophile.

Les fonctions esters *N*-hydroxysuccinimide, isocyanates et isothiocyanates peuvent également être forcées à réagir avec les fonctions alcool en plus des fonctions amine.

**Les fonctions réactives spécifiques des acides carboxyliques** sont présentes dans les résidus Asp, Glu et en partie C-terminale des protéines. Ces fonctions ne sont pas réactives en soi et nécessitent une activation.

Cette activation est généralement effectuée avec des carbodiimides. La *O*-acylurée formée par cette activation permet de réagir avec une amine primaire et de former une liaison amide.

Les fonctions réactives spécifiques des thiols sont les fonctions N-maléimide et disulfure.

La fonction thiol est la plus réactive des fonctions nucléophiles au sein d'une protéine. Cependant, les chaînes latérales des résidus cystéines sont souvent engagées dans des ponts disulfures ce qui empêche leur réaction avec des agents de réticulation chimique. Par conséquent, une réaction de réduction (avec de l'éthanedithiol ou EDT) de ces liaisons est généralement nécessaire afin de retrouver les fonctions thiols libres. Le pKa des thiols des résidus cystéines étant d'environ 8,6, leur réactivité augmente lorsque l'on forme l'ion thiolate à pH supérieur à 8,6.

**Les fonctions réactives spécifiques des guanidines** sont les composés 1,2-dicarbonyl qui réagissent spécifiquement avec les chaînes latérales des arginines.

**Les fonctions réactives non spécifiques** réagissent sur des molécules par exposition à la lumière UV. Un agent idéal photoréactif doit avoir différentes qualités :

- Il a une forte réactivité
- Il est capable de réagir indifféremment sur n'importe quel type de résidu
- Il est stable dans le noir
- Il réagit avec une lumière dont la longueur d'onde ne cause aucun dommage photolytique à l'échantillon biologique
- Le produit de sa réaction doit être stable.

Les mécanismes de réaction de ces fonctions sont généralement radicalaires ce qui permet d'agir indifféremment sur divers résidus. D'une manière générale, les agents de réticulation chimique ayant une fonction réactive non-spécifique possèdent aussi une fonction réactive spécifique. De cette manière, il est possible de cibler des résidus d'intérêt avec une première étape de fixation mettant en jeu la fonction réactive spécifique puis de marquer tous les résidus présents dans un certain périmètre (défini par la longueur du bras espaceur liant les deux fonctions réactives) grâce à l'action de la fonction réactive non-spécifique.

Il existe deux types de fonctions couramment utilisées dans ce but : les benzophénones et les arylazides.

PROCEDE DE PREPARATION

❖ **Fonctionnalisation de la matrice M**

Dans le cas où le support solide ne porte pas de linker, il convient d'ajouter L à la matrice. La fonctionnalisation d'un support par différents linkers est connue de l'homme du métier. Par exemple, le support solide fonctionnalisé pour-exposer des fonctions $-NH_2$ est fonctionnalisé par un couplage peptidique avec le linker qui possède une fonction acide carboxylique, éventuellement après déprotection ou neutralisation de sels d'ammonium. Le support solide peut présenter un halogène, comme le chlore, qui peut être substitué par un carboxylate ou un alcoolate présent sur le linker pour obtenir un lien ester ou éther entre le linker et la matrice. Le support solide peut présenter un halogène, comme le chlore, qui peut être substitué par un carboxylate ou un alcoolate présent sur le linker pour obtenir un lien ester ou ether entre le linker et la matrice.

❖ **Couplage entre le linker et le bras espaceur**

Le procédé de préparation de l'agent de réticulation de l'invention comprend les différentes réactions suivantes :

(i)

- éventuellement couplage peptidique entre la fonction amine du linker et la fonction acide carboxylique d'un acide aminé dont la fonction amine est protégée, puis déprotection de la fonction amine,

- éventuellement couplage par réaction d'estérification entre la fonction alcool du linker et la fonction acide carboxylique d'un acide aminé dont la fonction amine est protégée, puis déprotection de la fonction amine. On pourra utiliser par exemple la disiopropylcarbodimimide (DIC) en présence de 0.1 eq de diméthylaminopyridine (DMAP) pour réaliser cette estérification
- éventuellement couplage par réaction d'estérification entre la fonction halogène du linker et la fonction acide carboxylique sous forme de carboxylate d'un acide aminé dont la fonction amine est protégée, puis déprotection de la fonction amine. On pourra utiliser par exemple la TEA triéthylamine ou les sels de carbonate de césium pour générer le carboxylate de l'acide carboxylique à estérifier.
- éventuellement couplage peptidique entre la fonction acide du linker et la fonction amine d'une diamine dont une des fonctions amines peut être protégée, puis déprotection de la fonction amine,
- éventuellement couplage par formation d'ester entre la fonction acide du linker et la fonction alcool d'un amino alcool dont la fonction amine est protégée, puis déprotection de la fonction amine,
- éventuellement couplage peptidique entre la fonction amine du linker et un diacide ou un anhydride et
- éventuellement couplage peptidique entre la fonction amine du linker et un diacide dont une fonction est protégée sous forme d'ester et puis déprotection de la fonction acide protégée.
- éventuellement couplage par estérification entre la fonction alcool du linker et un diacide ou un anhydride et,
- éventuellement couplage par estérification entre la fonction alcool du linker et un diacide dont une fonction est protégée sous forme d'ester et puis déprotection de la fonction acide protégée.
- éventuellement couplage par réaction d'estérification entre la fonction halogène du linker et la fonction acide carboxylique sous forme de carboxylate d'un diacide dont une fonction est protégée sous forme d'ester puis déprotection de la fonction acide protégée.
- éventuellement couplage par réaction d'estérification entre la fonction halogène du linker et la fonction acide carboxylique sous forme de carboxylate d'un diacide

(ii) répétition une ou plusieurs fois des étapes suivantes afin d'obtenir le bras espaceur désiré

- couplage peptidique entre la fonction acide du composé obtenu à l'étape précédente et la fonction amine d'un acide aminé dont la fonction acide est protégée, puis déprotection de la fonction acide
- couplage peptidique entre la fonction amine du composé obtenu à l'étape précédente et la fonction acide carboxylique d'un acide aminé dont la fonction amine est protégée, puis déprotection de la fonction amine,
- couplage par réaction d'estérification entre la fonction alcool du composé obtenu à l'étape précédente et la fonction acide carboxylique d'un acide aminé dont la fonction amine est protégée, puis déprotection de la fonction amine. On pourra utiliser par exemple la disiopropylcarbodimimide (DIC) en présence de 0.1 eq de diméthylaminopyridine (DMAP) pour réaliser cette estérification
- couplage peptidique entre la fonction acide du composé obtenu à l'étape précédente et la fonction amine d'une diamine dont une des fonctions amines peut être protégée, puis déprotection de la fonction amine,
- couplage par formation d'ester entre la fonction acide du composé obtenu à l'étape précédente et la fonction alcool d'un amino alcool dont la fonction amine est protégée, puis déprotection de la fonction amine,
- couplage peptidique entre la fonction amine du composé obtenu à l'étape précédente et un diacide ou un anhydride et
- couplage peptidique entre la fonction amine du composé obtenu à l'étape précédente et un diacide dont une fonction est protégée sous forme d'ester et puis déprotection de la fonction acide protégée.
- couplage par estérification entre la fonction alcool du composé obtenu à l'étape précédente et un diacide ou un anhydride et,
- couplage par estérification entre la fonction alcool du composé obtenu à l'étape précédente et un diacide dont une fonction est protégée sous forme d'ester et puis déprotection de la fonction acide protégée.

L'homme du métier saura à l'aide de ses connaissances générales choisir les réactifs et réactants à mettre en présence pour obtenir par couplage(s) peptidique(s)un bras espaceur B tel que défini ci-dessus.

Lorsque la réaction de couplage est réalisée avec un acide carboxylique, celui-ci sera réalisé de préférence en présence d'un agent de couplage, tel que le diisopropylcarbodiimide (DIC), le dicyclohexylcarbodiimide (DCC), le chlorhydrate de 1-(3-dimethylaminopropyl)-3-éthylcarbodiimide (EDC), le carbonyldiimidazole (CDI), le 2-H-benzotriazole-1-yl)-1,1,3,3-tétraméthyluronium hexafluorophosphate (HBTU), le 2-(1H-benzotriazole-1-yl)-1,1,3,3-tétraméthyluronium tétrafluoroborate (TBTU) ou encore le O-(7-azobenzotriazol-1-yl)-1,1,3,3-tétraméthyluronium hexafluorophosphate (HATU), éventuellement associé à un auxiliaire de couplage tel que le *N*-hydroxy succinimide (NHS), le *N*-hydroxy benzotriazole (HOBt), le 3,4-dihydro-3-hydroxy-4-oxo-1,2,3-benzotriazole (HOOBt), le 1-hydroxy-7-azabenzotriazole (HOAt) ou le *N*-hydroxysylfosuccinimide (sulfo NHS).

Les réactions de protection/déprotection des fonctions amine et les réactions de couplage peptidique sont des réactions

bien connues de l'homme du métier.

❖ **Couplage de l'agent de réticulation A à l'extrémité du bras espaceur**

L'agent de réticulation A peut être fixé à B sous forme inactive ou protégée avant d'être activé ou déprotégé.

Dans le cas d'un bras monofonctionnel, soit A présente la formule (IIIbis), et X est une fonction ester NHS, la synthèse de l'agent de réticulation selon l'invention st par exemple terminée par activation de l'acide carboxylique terminal du bras espaceur B par le *N*-hydroxysuccinimide (HOSu).

Différentes réactions de couplage de l'agent de réticulation A à l'extrémité du bras espaceur sont décrites ci-dessous :

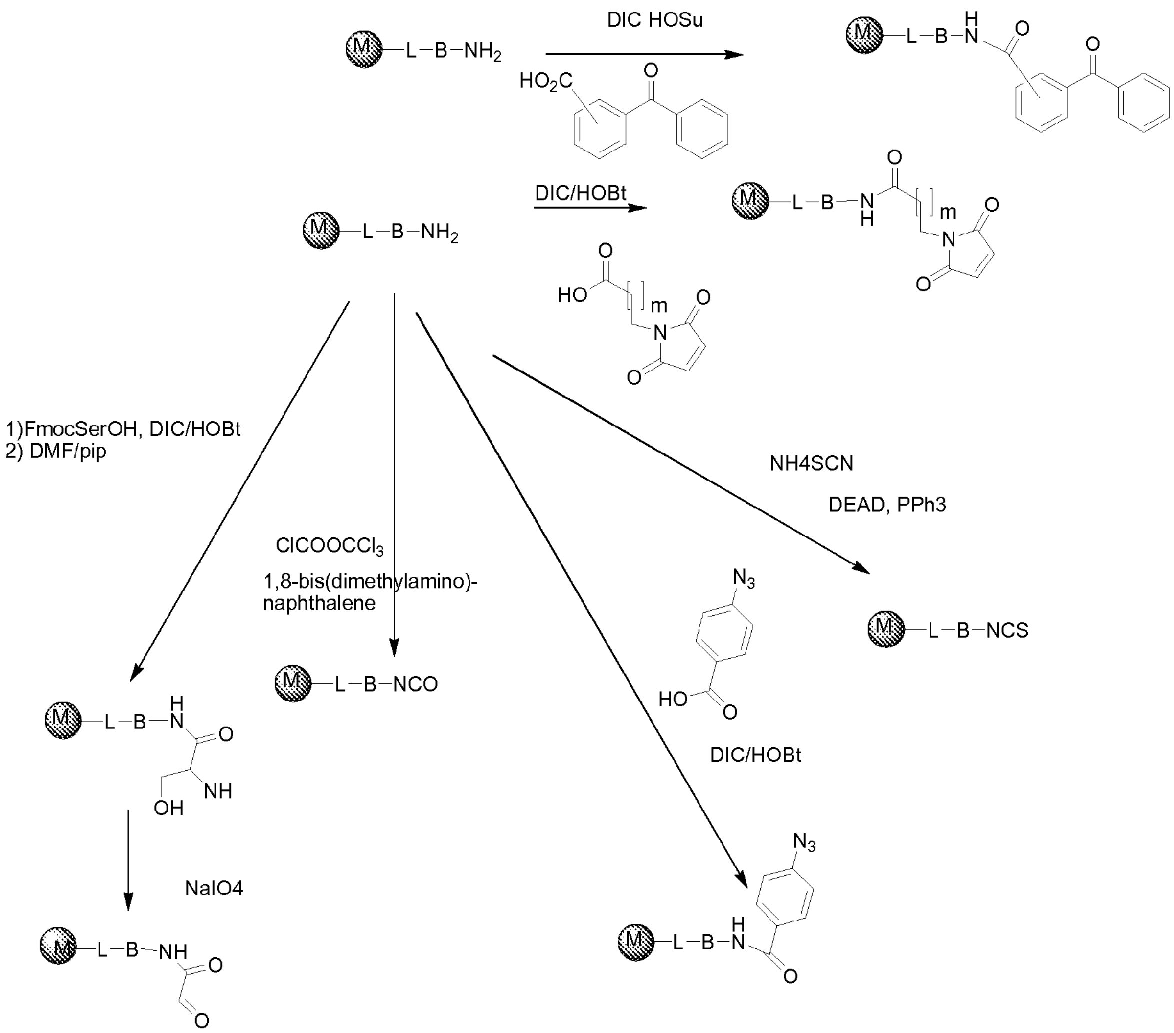

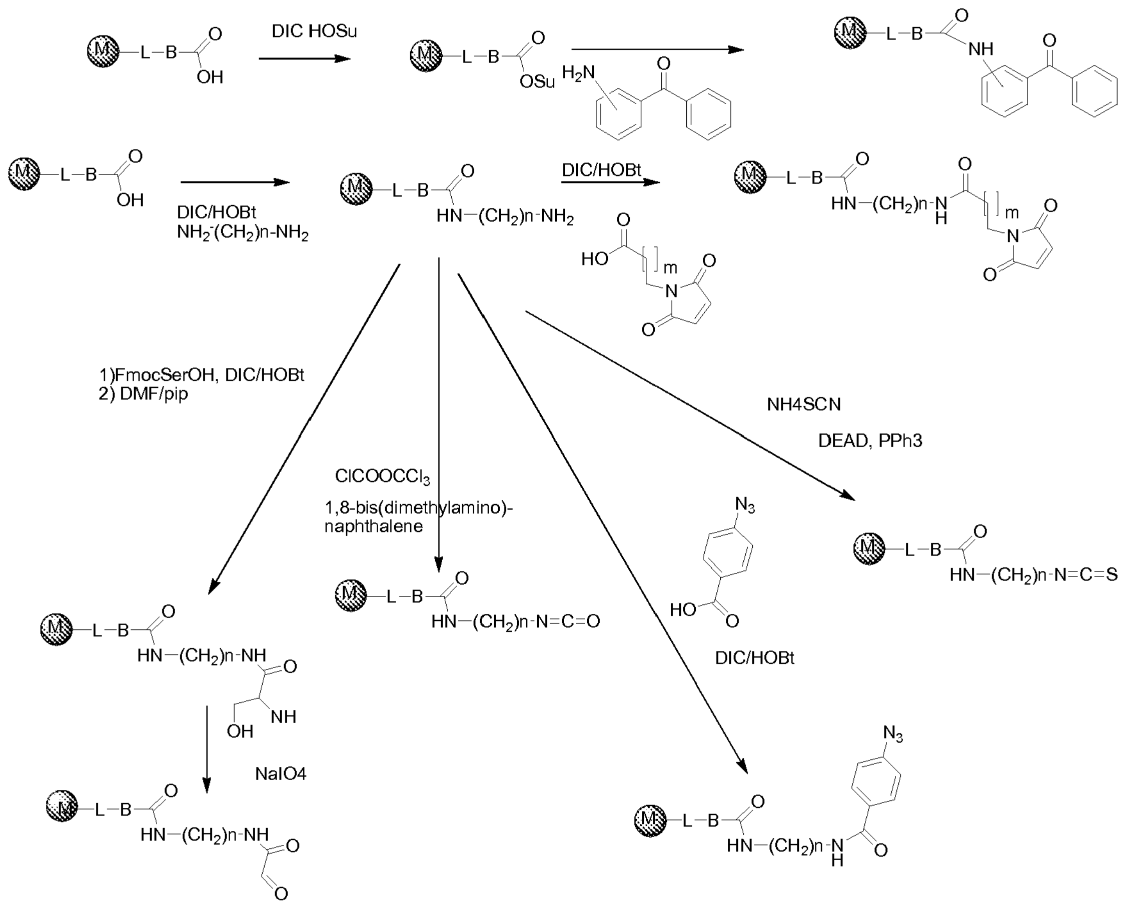


METHODE DE RETICULATION DE PROTEINES SUR SUPPORT SOLIDE POUR ANALYSE STRUCTURALE PAR SPECTROMETRIE DE MASSE

❖ **Réticulation de protéine sur support solide**

L'agent de réticulation chimique supporté est mis en présence de la protéine dissoute. Avantageusement, l'agent de réticulation chimique supporté est mis en présence de la protéine dissoute dans un tampon salin afin de s'approcher des conditions physiologiques.

Il en résulte une fixation covalente de la protéine sur le support solide comme dans le cas d'agents de réticulation chimique en solution par l'intermédiaire des fonctions réactives X et/ou X'

❖ **Lavages du support solide**

Le support solide est alors lavé, de préférence avec différents solvants, afin d'extraire du milieu les protéines n'ayant pas réagi. Une attention particulière doit être portée à la possibilité d'adsorption aspécifique de protéines sur le support solide.

Avantageusement, les lavages sont réalisés avec un ou plusieurs solvants aqueux ou organiques.

Avantageusement, les lavages sont réalisés avec un ou plusieurs solvants choisis dans le groupe constitué de tampon PBS, solution aqueuse de HCl, solution aqueuse de NaOH, eau, méthanol, DMF, DCM.

❖ **Détermination du taux de réticulation de la protéine**

Un aliquot de résine peut être utilisé afin de contrôler le taux de réticulation de la protéine. Pour cela, les protéines fixées sont remises en solution lors d'un clivage chimique entre le support solide et le bras espaceur B. Le milieu est alors analysé par spectrométrie de masse. La protéine modifiée doit être visible sur le spectre de masse. Si

les lavages ont été efficaces, peu ou pas de protéine non modifiée n'est détectée lors de l'analyse.

❖ **Digestion enzymatique sur support solide**

Les protéines réticulées et immobilisées sur le support solide sont digérées. Pour ce faire, le support solide est mis en présence d'une enzyme (*e.g*. la trypsine) solubilisée, de préférence dans un tampon salin.
Avantageusement, le support solide est compatible (*i.e*. taille de pores, hydrophilicité) avec l'utilisation d'une enzyme.

❖ **Lavages du support solide**

Une fois la digestion enzymatique effectuée, des fragments peptidiques réticulés sont immobilisés sur le support solide. Une série de lavages successifs est alors réalisée afin de retirer du milieu l'enzyme de digestion et surtout les peptides non modifiés qui n'ont pas été immobilisés de manière covalente sur le support solide.
Avantageusement, les lavages sont réalisés avec un ou plusieurs solvants aqueux ou organiques.
Avantageusement, les lavages sont réalisés avec un ou plusieurs solvants choisis dans le groupe constitué de tampon PBS, solution aqueuse de HCl, solution aqueuse de NaOH, eau, méthanol, DMF, DCM.

❖ **Clivage**

Les peptides d'intérêt réticulés sont libérés en solution après le clivage chimique entre le support solide M et le bras espaceur B, éventuellement entre le linker L et le bras espaceur B si le support est fonctionnalisé par un linker. Un mélange contenant majoritairement voire exclusivement des peptides réticulés est alors obtenu et peut être analysé par spectrométrie de masse.

❖ **Analyse par spectrométrie de masse**

SPECTROMETRIE DE MASSE

De préférence, l'analyse par spectrométrie de masse est effectuée par spectrométrie MALDI - TOF.
Les échantillons sont déposés soit en goutte séchée, soit sur couche mince.
Le dépôt en goutte séchée est préféré. Il est réalisé par co-cristallisation d'une goutte de solution matricielle et d'une goutte d'une solution d'analyte. Les solutions matricielles préférées sont HCCA (acide $\alpha$-cyano 4-hydroxycinnamique) pour l'analyse de peptides et AS (acide sinapinique) pour l'analyse de protéines.

LOGICIEL

Avantageusement, les données de l'analyse par spectrométrie de masse des mélanges de peptides sont analysées par un logiciel.
En effet, l'analyse par spectrométrie de masse des mélanges de peptides fournis par l'étape de digestion enzymatique apporte généralement une quantité importante de données. Afin de faciliter l'identification des peptides détectés lors de ces expériences, de nombreux logiciels de prédiction ont été créés (*e.g*. Pro-CrossLink, VirtualMSLab, SearchXLink et MS2Assign).
Le but de ces logiciels est de fournir rapidement une liste de peptides potentiels non modifiés ou modifiés par l'agent de réticulation utilisé, correspondant à chaque valeur m/z du spectre de masse. Les valeurs expérimentales sont comparées aux valeurs théoriques calculées à partir d'une digestion *in silico* du modèle protéique étudié.
Dans le cadre de la présente invention, un logiciel de prédiction, appelé MSX-3D, a été créé au sein de l'Institut de Biologie et de Chimie des Protéines (IBCP) à Lyon.

Figures

- Figure 1 : Schéma représentant la méthode de réticulation des protéines sur support solide pour analyse structurale par spectrométrie de masse
- Figure 2 : Support solide avec agent monofonctionnel.
- Figure 3 : Spectre de masse du test d'immobilisation du peptide 34 avec la résine 26.
- Figure 4 : Spectre de masse du test d'immobilisation du cytochrome c sur la résine 24.

Figure 5 : Spectre de masse du test d'immobilisation du peptide 34 avec la résine 29.
Figure 6 : Spectre de masse du test d'immobilisation du cytochrome c sur la résine 29.
Figure 7 : Spectre de masse du test d'immobilisation du cytochrome c sur la résine 31.
Figure 8 : Spectre de masse de la digestion du cytochrome c avec de la trypsine (avec matrice HCCA).
Figure 9 : Spectre de masse de la digestion trypsique du cytochrome c immobilisé sur la résine 31 (avec matrice HCCA) : ★ signal de peptide modifié.

Les exemples suivants illustrent l'invention sans en limiter la portée.

## Exemple 1 : Bras monofonctionnels sur support solide

### I-I. Synthèse

Un linker de type Rink Amide a été placé entre le support solide et le bras monofonctionnel afin de pouvoir libérer en solution l'analyte fixé. La fonction réactive ester NHS a été choisie afin de réagir de manière spécifique avec les amines primaires (*i.e.* N-terminales et N-ε des lysines) présentes dans les protéines (voir Figure 2).

Les supports solides utilisés sont les suivants :

- Lanterne Fmoc-Rink Amide-SynPhase-PS (série D, 35 μmole) : Mimotopes
- Lanterne Fmoc-Rink Amide-SynPhase-PA (série D, 18 μmole) : Mimotopes
- Résine Fmoc-Rink Amide-aminométhyl-polystyrène (100-200 mesh, 0.4-0.9 mmol/g) : Iris-Biotech
- Résine EAH Sepharose™ 4B (90 μm, 7-12 μmol/mL de matrice drainée) : GE Healthcare
- Résine aminométhyl-ChemMatrix (100-200 mesh, 0,8-1,0 mmol/g) : Iris-biotech
- Aminométhyl-méthacrylate Synbeads (150-300 μm, 0,7-1,0 mmol/g): Iris-Biotech
- Résine Fmoc-Rink Amide-NovaGel (100-200 mesh, 0,7 mmol/g) : Novabiochem
- Résine cystéamine-2-chlorotrytil (100-200 mesh, 1,25 mmol/g) : Novabiochem
- Résine aminométhyl-PL-PEGA (150-300 μm, 0,4 mmol/g): Polymer Laboratories.

Le groupement protecteur temporaire des amines N-α est l'uréthane de type 9-fluorénylméthyloxycarbonyle (Fmoc). Ce groupement est déprotégé en présence d'amine secondaire (pipéridine).

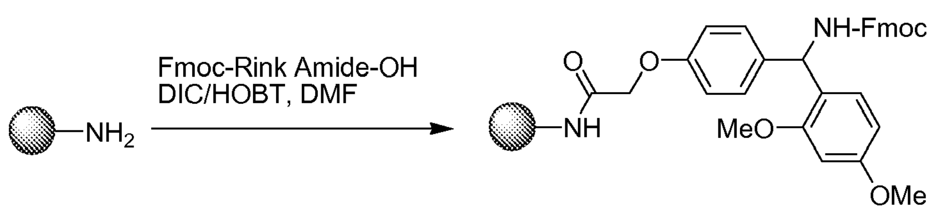


**Schéma 1 :** Couplage de l'acide Fmoc-Rink Amide-OH sur une amine primaire du support solide.

Après avoir ôté la protection Fmoc, le linker Rink Amide permet l'ancrage d'un aminoacide sur le côté C-terminal en créant une liaison amide. Il est clivable en condition acide avec de l'acide trifluoroacétique (TFA) et libère un peptide C-terminal amidé.

Après déprotection de la fonction amine primaire du linker Rink Amide, différents bras espaceurs ont été couplés au linker.

1-1- Méthode de dosage du groupement Fmoc

Ce dosage par spectrométrie UV permet d'évaluer la charge (en mmol/g) d'une résine possédant un groupement 9-Fluorénylméthoxycarbonyle (Fmoc) - *e.g.* Fmoc-Rink Amide, Fmoc-aminoacide. Cette méthode consiste à déprotéger une quantité donnée de résine et de doser en solution la 1(9-fluorénylméthyl)pipéridine, produit de la réaction entre la pipéridine et le dibenzofulvène libéré par le Fmoc (cf. Schéma 2). Cette mesure donne une valeur en mmoles par gramme de résine.

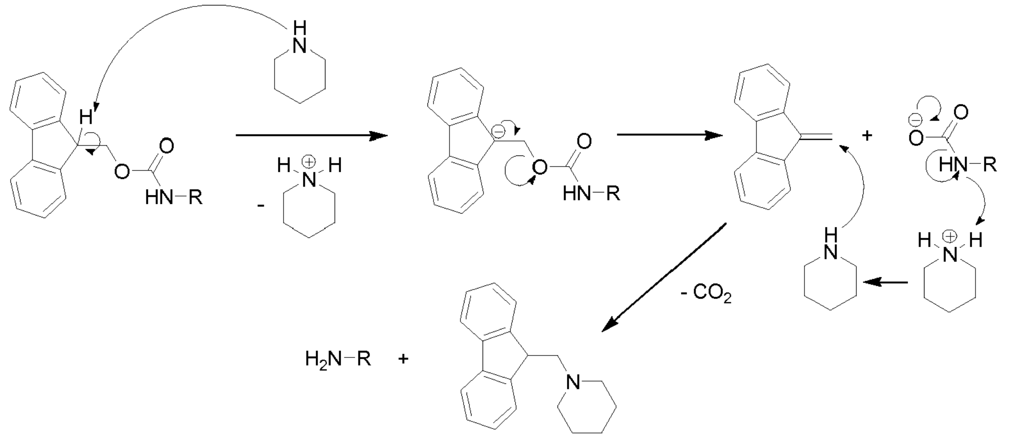


1(9-fluorénylméthyl)pipéridine

**Schéma 2 :** Déprotection de l'amine N-Fmoc.

Pour évaluer cette charge, le protocole suivant a été utilisé :

- 10 mg de résine sont placés dans un tube à hémolyse
- 1 mL d'une solution de pipéridine/DMF (20 : 80) sont ajoutés dans le tube
- Le tube est agité pendant 30 minutes
- 100 $\mu$L du milieu réaction sont prélevés et dilués dans 10 mL de DMF
- L'absorbance du milieu à une longueur d'onde de 301 nm est déterminée avec un spectromètre UV, après avoir effectué un blanc avec du DMF.

La charge de la résine est calculée suivant l'équation (déduite de la loi de Beer - Lambert) :

$$\text{Charge (mmol/g de résine)} = (1000 \times A_{301})/(m \times 7800 \times d)$$

Avec : $A_{301}$ : absorbance à 301 nm
m : masse de résine pesée en mg
d : largeur de la cuve (égale à 0,01 cm).

<u>I-1-2 - Protocole de synthèse peptidique sur résine Fmoc-Rink Amide en stratégie Fmoc (Fmoc - SPPS)</u>

*I-1-2- a - Déprotection de la résine Fmoc-Rink Amide*

Le groupement Fmoc du linker Rink Amide est retiré par réaction avec mélange DMF/pipéridine (80 : 20) pendant 2 fois 20 minutes. La résine est lavée successivement (3 fois 2 minutes pour chaque solvant) avec les solvants suivants : DMF, méthanol, DCM. La résine est ensuite séchée sous vide.

*I-1-2- b - Couplage d'un Fmoc-aminoacide*

Les couplages des aminoacides ont été réalisés dans du DMF par activation de la fonction acide carboxylique avec de l'HBTU en présence d'une amine tertiaire (NMM) selon le protocole suivant :
A un volume de solution de Fmoc-aminoacide à 300 mM sont ajoutés un volume d'une solution de NMM à 600 mM et un volume d'une solution de HBTU à 300 mM. Cette solution est alors versée dans le réacteur contenant la résine puis placée sous agitation pendant 1 heure. La résine est lavée successivement (3 fois 2 minutes pour chaque solvant) avec les solvants suivants : DMF, méthanol, DCM. La résine est ensuite séchée sous vide.
*N.B. : Le volume total de solvant utilisé pour la réaction dépend de la quantité de résine. Par exemple : pour 100 mg*

*de résine à 0,5 mmol/g, un volume total de 2 mL de solvant est utilisé (soit 12 équivalents de Fmoc-aminoacide par fonction amine primaire sur la résine)*.

*I-1-2-c - Déprotection d'un Fmoc-aminoacide supporté*

Le groupement Fmoc de l'aminoacide supporté est retiré par réaction avec un mélange DMF/pipéridine (80 : 20) pendant 20 minutes. La résine est lavée successivement (3 fois 2 minutes pour chaque solvant) avec les solvants suivants : DMF, méthanol, DCM. La résine est ensuite séchée sous vide.

**I-2 Synthèse des bras monofonctionnels sur résine Rink-Amide**

**Tableau 1** : Bras monofonctionnels synthétisés sur différents supports solides.

| Bras monofonctionnel | | Support solide | Référence |
|---|---|---|---|
| 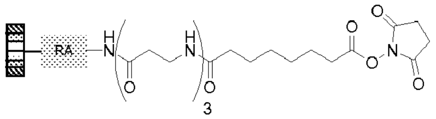 | | SynPhase-PA (18 µmol/u) | Lanterne **13** |
| 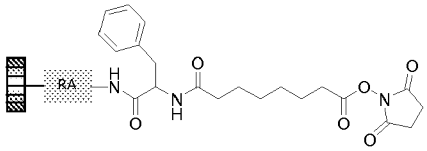 | | SynPhase-PA (18 µmol/u) | Lanterne **14** |
| | | SynPhase-PS (35 µmol/u) | Lanterne **15** |
| 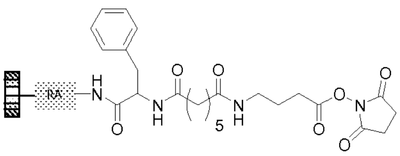 | | SynPhase-PA (18 µmol/u) | Lanterne **16** |
| | | SynPhase-PS (35 µmol/u) | Lanterne **17** |
| 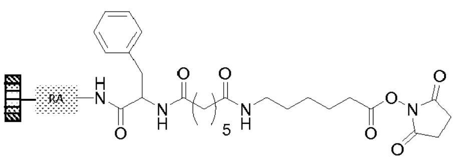 | | SynPhase-PA (18 µmol/u) | Lanterne **18** |
| | | Tentagel (0,2 mmol/g) | Résine **19** |
| 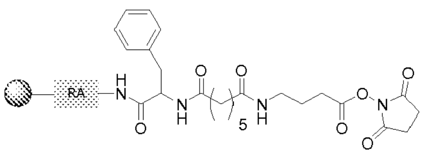 | | Novagel (0,7 mmol/g) | Résine **20** |
| | | Tentagel (0,2 mmol/g) | Résine **21** |
| 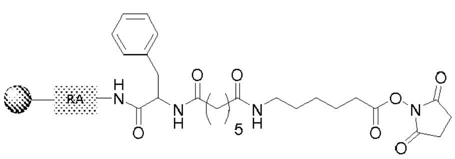 | | Novagel (0,7 mmol/g) | Résine **22** |
| 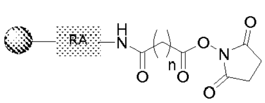 | n = 1 | PEGA (0,4 mmol/g) | Résine **23** |
| | n=2 | | Résine **24** |
| | n=3 | | Résine **25** |
| | n=4 | | Résine **26** |
| | n=5 | | Résine **27** |
| 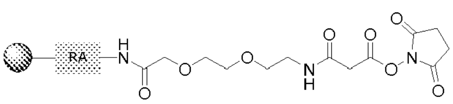 | | PEGA (0,4 mmol/g) | Résine **28** |

(suite)

| Bras monofonctionnel | Support solide | Référence |
|---|---|---|
| 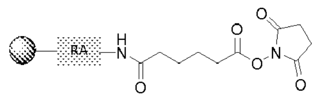 | Sepharose (7-12 μmole/mL de matrice drainée) | Résine **29** |
| 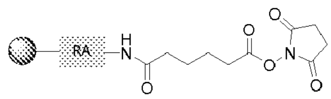 | ChemMatrix (0,8 mmol/g) | Résine **30** |
| 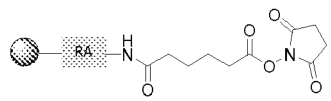 | Synbeads (0,7 mmol/g) | Résine **31** |

L'influence de la longueur (*e.g.* acide malonique en $C_3$, acide octanedioique en $C_8$) et de la nature (*e.g.* hydrophile avec PEG) du bras espaceur ont été étudiées.

Les diacides carboxyliques (malonique, adipique, heptanedioique et octanedioique) ont été couplés sur les résines fonctionnalisées à des amines primaires grâce à une activation d'une de leurs fonctions acides avec un mélange de DIC et de HOBt dans du DMF (cf. Schéma **3**).

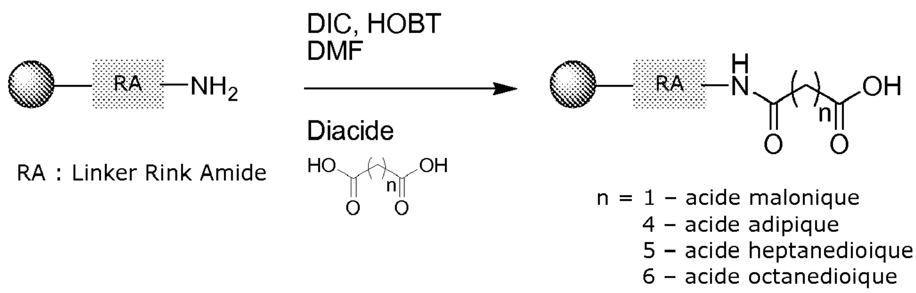


**Schéma 3 :** Couplage de l'acide malonique, adipique, heptanedioique et octanedioique.

Les anhydrides succinique et glutarique ont été dissous dans du DMF afin d'effectuer leur couplage sur les fonctions amines primaires du support solide (cf. Schéma 4).

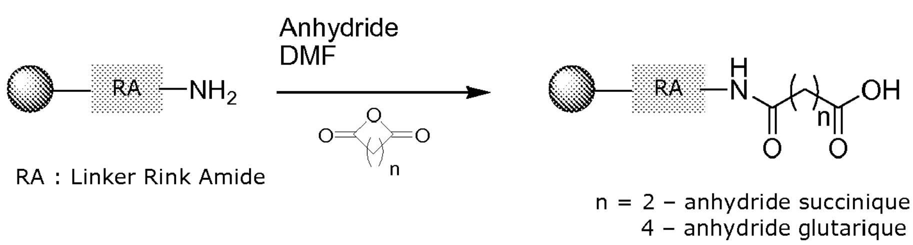


**Schéma 4 :** Couplage des anhydrides succinique et glutarique.

Les acides 4-aminobutyrique et 6-aminohexanoique ont été couplés à des fonctions acides carboxyliques pré-activées sur support solide à l'aide d'un mélange de DIC et HOBt dans du DMF (cf. Schéma **5**).

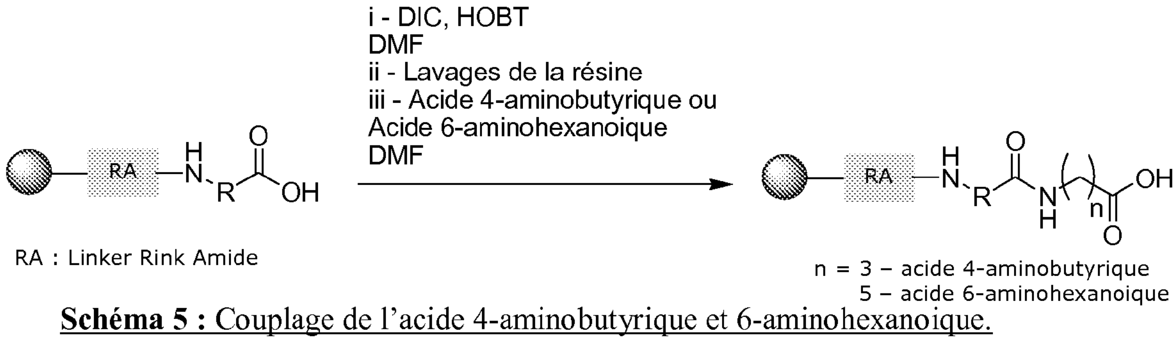


**Schéma 5 :** Couplage de l'acide 4-aminobutyrique et 6-aminohexanoique.

La β-alanine a été couplée à des fonctions amines primaires sur support solide grâce à une activation de l'acide Fmoc-β-alanine avec un mélange de DIC et d'HOBt dans du DMF (cf. Schéma **6**). Une déprotection de l'amine primaire avec une solution de DMF/pipéridine a ensuite été réalisée afin de permettre le couplage d'autres réactifs possédant une fonction acide carboxylique (*e.g.* acide adipique).

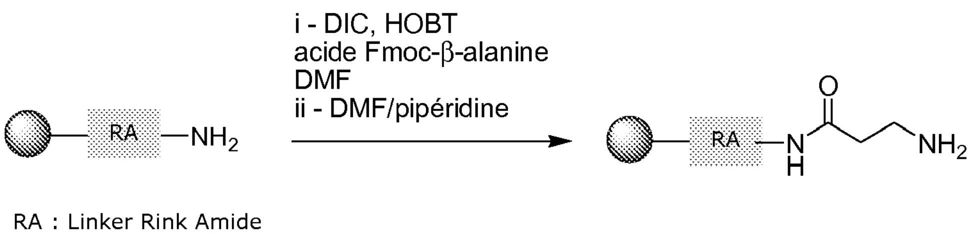


**Schéma 6 :** Couplage de l'acide Fmoc-β-alanine sur support solide.

La phénylalanine N-Fmoc protégée a été couplée sur support solide grâce à une activation de son acide carboxylique avec un mélange de DIC et d'HOBt dans du DMF (cf. Schéma **7**). Par la suite, le groupement Fmoc protégeant l'amine primaire a été enlevé avec une solution de DMF/pipéridine afin de continuer la synthèse des bras mono fonctionnels.

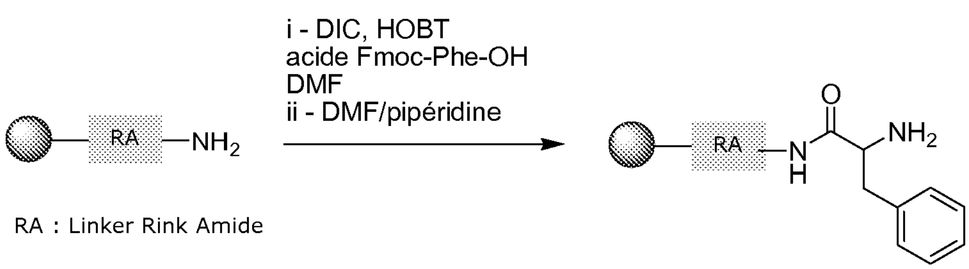


**Schéma 7 :** Couplage de l'acide Fmoc-Phe-OH sur support solide.

L'acide 8-(Fmoc-amino)-3,6-dioxaoctanoique ou Fmoc-$O_2$O-OH a été utilisé et couplé à des fonctions amines primaires sur le support solide en activant sa fonction acide carboxylique avec un mélange de DIC et d'HOBt dans du DMF. La protection Fmoc de l'amine primaire a été retirée avec un mélange de DMF et de pipéridine afin de coupler d'autres réactifs pour l'élaboration de bras monofonctionnels.

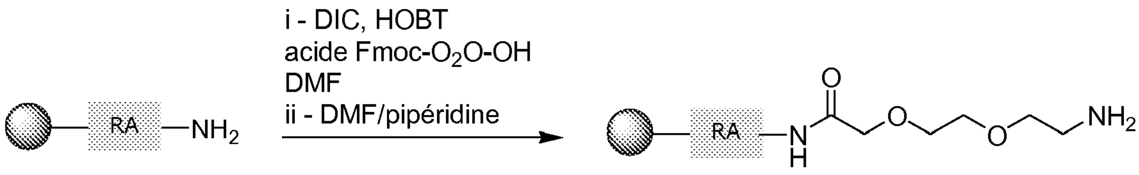


RA : Linker Rink Amide

**Schéma 8 :** Couplage de l'acide 8-(Fmoc-amino)-3,6-dioxaoctanoique.

Enfin, chaque bras monofonctionnel synthétisé sur support solide est terminé par une fonction ester NHS. Pour cela, l'activation de l'acide carboxylique terminal a été effectuée à l'aide d'un mélange de DIC et de *N*-hydroxysuccinimide (HOSu) dans du DMF (cf. Schéma **9**).

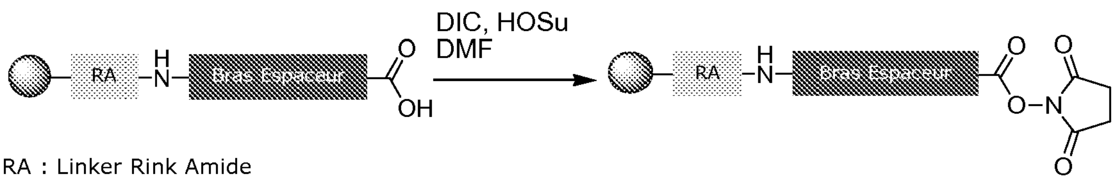


RA : Linker Rink Amide

**Schéma 9 :** Activation des fonctions acides carboxyliques des bras espaceurs en ester NHS.

2 - Tests d'immobilisation de différents analytes

Pour chaque support solide préparé, des tests d'immobilisation de différentes molécules ont été effectués afin d'évaluer le meilleur système « support solide/bras espaceur ». Trois types de molécules ont été utilisées : la benzylamine, des peptides (possédant une ou plusieurs amines primaires libres), le cytochrome c de coeur de cheval.

2 - 1 *Protocoles des expériences d'immobilisation*

❖ **Tests de fixation de la benzylamine**

La benzylamine a été utilisée afin de vérifier l'activation correcte de l'acide en bout de bras espaceur en ester NHS.

Pour cela, 50 mg de résine ou une tranche de Lanterne ont été ajoutés à 1 mL d'une solution de benzylamine à 100 $\mu$M dans du DMF pendant 1 heure. La résine ou la Lanterne a été lavée avec différents solvants (DMF, méthanol, DCM) et séchée sous vide. Le linker Rink Amide a été clivé avec une solution de TFA pendant 30 minutes. Après filtration, la solution de clivage a été évaporée avec un flux d'azote et le résidu a été repris avec un mélange de eau/ACN/TFA (50 : 50 : 0,1).

La solution obtenue a été analysée par LC/MS - ESI en mode positif.

❖ **Tests de fixation de peptides**

Différents peptides ont été synthétisés afin d'évaluer l'efficacité de l'immobilisation sur les différents supports solides en milieu aqueux (*i.e.* tampon PBS à pH 7,5).

Les peptides qui ont été utilisés sont : H-AEKLKEYLKK-$NH_2$ (Peptide **32**)
H-AGEPKLDAGV-$NH_2$ (Peptide **33**)
H-RKGKLAF-$NH_2$ (Peptide **34**).

Tous ces peptides possèdent au moins deux sites de fixation possible (N-terminal ou N-ε-lysine).

Ces tests ont été réalisés en mélangeant 50 mg de résine ou une tranche de Lanterne à 1 mL d'une solution de peptide modèle à 2,5 mM dans un tampon PBS à pH 7,5 pendant 1 heure. Le support solide a été ensuite lavé avec différents solvants (tampon PBS, solutions aqueuses de HCl à 1N et NaOH à 1N, eau, méthanol, DMF, DCM) et séché sous vide. Le clivage du linker Rink Amide a été effectué avec une solution de TFA pendant 30 minutes. Après filtration du support solide, le solvant a été évaporé avec un flux d'azote. Le résidu sec a été dissous avec une solution d'eau/ACN/TFA (50 : 50 : 0,1).

La solution a été analysée par LC/MS - ESI ou par spectrométrie de masse MALDI-TOF avec de la matrice HCCA.

❖ **Tests de fixation du cytochrome c de coeur de cheval**

Le cytochrome c est un bon modèle pour les tests d'immobilisation sur support solide.

- Sa bonne solubilité dans les tampons aqueux, tel que les tampons PBS, permet de pouvoir l'utiliser dans de hautes concentrations afin d'optimiser le taux de fixation sur le support solide.
- Sa coloration rouge permet de visualiser directement s'il a été fixé ou adsorbé sur le support solide dans le cas où ce dernier est blanc (*e.g.* Synbeads).
- Enfin, sa structure cristalline étant connue, des comparaisons des données obtenues peuvent être faites avec les données structurales (*e.g.* accessibilité au solvant, contraintes de distances - dans le cas d'agents de réticulation).

Les tests de fixation du cytochrome c ont été réalisés en mélangeant 50 mg de résine ou une tranche de Lanterne avec 1 mL de solution de cytochrome c à 100 μM dans du tampon PBS à pH 7,5. Plusieurs lavages du support solide ont été effectués avec différents solvants (tampon PBS, solutions aqueuses de HCl à 1N et NaOH à 1N, eau, méthanol, DMF, DCM). Le support solide a été séché sous vide puis clivé avec une solution de TFA pendant 30 minutes. Après filtration du support solide, le solvant a été évaporé avec un flux d'azote puis le résidu sec a été dissous avec une solution d'eau/ACN/TFA (50 : 50 : 0,1).

La solution a été analysée par spectrométrie de masse MALDI-TOF avec de la matrice acide sinapinique.

2 - 2 *Résultats et commentaires des expériences d'immobilisation*

Les résultats des différents tests réalisés avec ces supports solides sont présentés dans le tableau **2** suivant.

**Tableau 2** : Résultats des tests d'immobilisation (👍 composé détecté, 👎 composé non détecté, ☝ composé détecté mais résultat ambigu).

| Support solide | Support solide | Benzylamine | Peptides modèles | | | Cytochrome c |
|---|---|---|---|---|---|---|
| | | | Peptide **32** | Peptide **33** | Peptide **34** | |
| Lanterne **13** | SynPhase-PA | 👍 | 👎 | | | 👎 |
| Lanterne **14** | SynPhase-PA | 👍 | 👎 | | | 👎 |
| Lanterne **15** | SynPhase- PS | 👍 | 👎 | | | 👎 |
| Lanterne **16** | SynPhase-PA | 👍 | 👎 | | | 👎 |
| Lanterne **17** | SynPhase-PS | 👍 | 👎 | | | 👎 |
| Lanterne **18** | SynPhase-PA | 👍 | 👎 | | | 👎 |
| Résine **19** | Tentagel | 👍 | 👎 | | | 👎 |

(suite)

| Support solide | Support solide | Benzylamine | Peptides modèles | | | Cytochrome c |
|---|---|---|---|---|---|---|
| | | | Peptide **32** | Peptide **33** | Peptide **34** | |
| Résine **20** | Novagel | 👍 | 👎 | | | 👎 |
| Résine **21** | Tentagel | 👍 | 👎 | | | 👎 |
| Résine **22** | Novagel | 👍 | 👎 | | | 👎 |
| Résine **23** | PEGA | 👍 | | 👎 | | 👎 |
| Résine **24** | PEGA | 👍 | | 👍 | | ☝ |
| Résine **25** | PEGA | 👍 | | 👍 | | 👎 |
| Résine **26** | PEGA | 👍 | | 👍 | 👍 | ☝ |
| Résine **27** | PEGA | 👍 | | 👍 | | 👎 |
| Résine **28** | PEGA | 👍 | | 👎 | | 👎 |
| Résine **29** | Sepharose | 👍 | | | 👍 | 👍 |
| Résine **30** | ChemMatrix | 👍 | | | 👍 | 👎 |
| Résine **31** | Synbeads | 👍 | | | 👍 | 👍 |

Plusieurs conclusions peuvent être tirées de ces résultats.

❖ **Les supports de type polystyrène sont incompatibles avec le tampon aqueux**

Aucun support solide dont la matrice est de type polystyrène n'a pu piéger ni le peptide modèle ni le cytochrome c (Lanternes **15, 17** et résines **19, 20, 21, 22**). Les Lanternes polyamide (Lanternes **13, 14, 16** et **18**) n'ont pas donné de meilleurs résultats que les Lanternes polystyrène. Ces échecs peuvent être expliqués par une incompatibilité de ces supports solides avec le tampon aqueux et ne sont pas dus à une mauvaise réactivité des esters NHS qui ont pu réagir efficacement avec la benzylamine.

❖ **Présence d'une réaction secondaire avec les bras espaceurs aminobutanoiques**

Les tests de fixation effectués sur les Lanternes **15, 16** et les résines **19, 20** ont mis en évidence une réaction secondaire récurrente. Cette réaction a été identifiée comme étant une cyclisation intramoléculaire produite lors de l'activation du bras monofonctionnel en ester NHS. Elle est favorisée par la formation d'un cycle lactame à 5 centres très stable (cf. Schéma **10**).

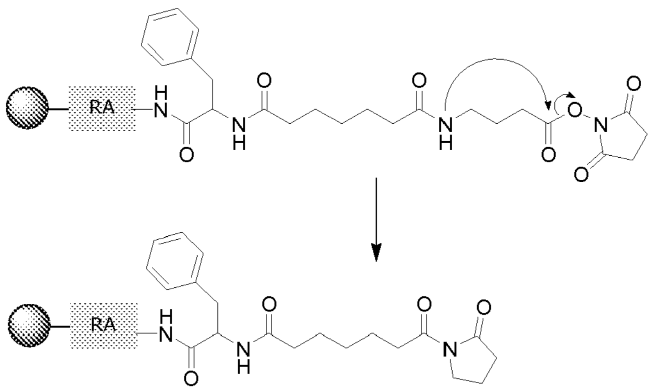

**Schéma 10 :** Réaction secondaire de cyclisation intramoléculaire.

❖ **Les supports de types PEGA (résines 24, 25, 26 et 27) ont pu immobiliser les peptides modèles**

La figure **3** montre le spectre de masse obtenu après immobilisation du peptide **34** sur la résine **26.** Ce spectre témoigne tout d'abord que le lavage de la résine a été efficace. En effet, l'ion moléculaire qui correspond au peptide **34** non modifié (m/z : 817,5 Da) n'est pas détecté. D'autre part, les signaux des trois possibilités de fixation du peptide **34** (correspondant aux trois amines primaires - N-terminal, deux N-ε de lysines) ont pu être détectés : une fixation simple avec un bras mono fonctionnel (m/z : 945,8 Da), une double fixation avec deux bras monofonctionnels (m/z : 1072,8 Da) et une triple fixation avec trois bras monofonctionnels (m/z : 1199,9 Da). Le taux de fonctionnalisation de la résine (0,4 mmol/g) est de tout évidence, suffisamment élevé pour permettre une triple réaction sur le peptide modèle.

Il est donc possible d'utiliser la résine PEGA et un bras espaceur monofonctionnel possédant une fonction ester NHS afin d'immobiliser un peptide dans un tampon PBS.

Malheureusement, les tests de fixation avec les résines **24** et **26** ont montré une forte adsorption non spécifique du cytochrome c car on retrouve la protéine non modifiée dans le spectre de masse avec les résines PEGA (cf. Figure **4**).

Bien qu'infructueuses, les expériences de fixation du cytochrome c ont mises en évidence l'importance du lavage de la résine. Il est important de limiter ou d'éliminer les phénomènes d'adsorption non spécifique de protéines dans la matrice.

Dès lors, différents systèmes de solvants ont été utilisés afin d'optimiser cette étape de lavage du support solide. Cette optimisation a été effectuée en réalisant des expériences contrôles avec du cytochrome c sur des supports solides non activés en esters NHS. Ces expériences ont été réalisées dans les mêmes conditions que celles effectuées avec des supports solides activés en esters NHS. L'utilisation d'une solution aqueuse acide (HCl 1N) et basique (NaOH 1N) a permis de laver de manière très efficace les résines. En effet, ces lavages permettent notamment de rompre les liaisons non covalentes entre la protéine et le support solide. L'utilisation de solvants organiques (*e.g.* DMF, méthanol) permet de laver les supports solides des produits organiques résiduels, tels que le *N*-hydroxysuccinimide résultant de la réaction d'un ester NHS avec une amine.

Seule la fixation du peptide **34** a pu être observée avec la résine **30** de type ChemMatrix®. Une observation commune peut être faite sur l'utilisation des matrices PEGA et ChemMatrix®.

❖ **La résine Sepharose : un support prometteur**

La fixation du peptide **34** a pu être observée sur la Sepharose (résine **29)** en spectrométrie de masse MALDI-TOF (cf. Figure **5**). De la même manière que pour les résines **24, 25, 26** et **27,** les trois possibilités de fixation du peptide ont pu être observées. Ceci montre que le support solide possède une forte concentration en bras monofonctionnel lui permettant de fixer jusqu'à trois fois le même peptide. D'autre part, aucun signal de peptide non modifié n'a été observé.

L'analyse du test de fixation du cytochrome avec la résine **29** présentée dans la figure **6** montre la présence de plusieurs signaux pouvant correspondre aux produits de la fixation de 2 à 6 bras monofonctionnels sur le cytochrome c. L'étape de lavage du support solide a permis de retirer entièrement l'excès de protéine dans le milieu car aucun signal correspondant à celle-ci n'est visible sur le spectre.

Bien que les résultats obtenus avec cette résine **29** soient encourageants, la matrice Sepharose montre une

dégradation due aux différents solvants organiques utilisés durant les réactions et les lavages.

❖ **La résine Synbeads macroporeuse est adaptée**

La résine Synbeads, quant à elle, a permis de répondre à tous les problèmes soulevés par les autres types de résines. Sa rigidité, permettant une manipulation aisée, et sa compatibilité avec les différents solvants organiques et aqueux ont pu être vérifiées. La résine **31,** élaborée avec de la résine Synbeads, a permis de fixer de manière covalente le peptide modèle **34** et le cytochrome c car elle possède une grande surface accessible quel que soit le solvant utilisé grâce à sa structure macroporeuse rigide.

L'adsorption non spécifique du cytochrome c sur la résine a tout d'abord été vérifiée avec une résine possédant le même type de bras espaceur mais non activé en ester NHS. La coloration rouge de la résine due à la fixation du cytochrome c a permis de visualiser l'efficacité des lavages de la résine. Après lavages, la résine **31** activée en ester NHS ayant réagi avec une solution de cytochrome c reste colorée en rose alors qu'elle devient incolore lorsqu'elle n'a pas été activée en ester NHS.

Le spectre de masse de l'expérience d'immobilisation du cytochrome c, présenté dans la figure 7, montre que la protéine a été fixée de manière covalente de 1 à 6 fois avec le support solide. Ce spectre montre des signaux de bonnes intensités et avec une résolution correcte.

Cette expérience montre que la résine **13** avec une charge de 0,7 mmol/g possède une densité de bras monofonctionnels suffisamment importante pour pouvoir réagir plusieurs fois sur la même protéine.

La figure **8** présente le spectre de masse obtenu lors de l'analyse en spectrométrie de masse MALDI-TOF, effectuée avec la matrice HCCA en dépôt goutte séchée, d'un mélange peptidique issu de la digestion trypsique du cytochrome c non modifié. Cette analyse sert de référence pour les résultats d'analyse des expériences de digestion sur support solide effectuées.

Un échantillon du test d'immobilisation du cytochrome c sur la résine **31** a ensuite été digéré avec de la trypsine directement sur le support solide. La résine a ensuite été lavée puis clivée avec une solution de TFA. Le résidu sec récupéré après filtration de la résine et évaporation du TFA a été analysé par spectrométrie de masse MALDI-TOF avec la matrice HCCA. Le spectre obtenu est présenté sur la figure **9**.

Le spectre de masse obtenu montre **16** nouveaux signaux de bonne résolution non détectés dans les spectres de masse du cytochrome c non modifié digéré avec de la trypsine. De plus, aucun signal de peptide non modifié n'est détecté ce qui montre l'efficacité de l'étape de lavage de la résine après digestion enzymatique.

L'analyse avec le logiciel MSX-3D de la liste d'ions détectés montre que chacun des signaux correspond à un peptide possédant au moins une lysine modifiée par un bras monofonctionnel. La digestion enzymatique ainsi que l'étape de lavage de la résine ont permis d'obtenir un mélange de peptides exclusivement modifiés.

Les résultats de ces prédictions sont présentés dans le tableau **3** ci-dessous.

**Tableau 3** : Liste des masses expérimentales et prédites de peptides modifiés par l'immobilisation sur support solide.

| [M + H]$^+$ Expérimental | Calculé | Déviation (ppm) | Séquence | Nombre de modification | Lysines modifiées |
|---|---|---|---|---|---|
| 1560,87 | 1560,85 | -14 | 26 - 38 | 1 | 27 |
| 1688,98 | 1688,94 | -25 | 26 - 39 | 1 | 27 |
| 1734,03 | 1733,99 | -23 | 87 - 99 | 1 | 87, 88 |
| | 1733,99 | -23 | 88 - 100 | 1 | 88,99 |
| 1803,04 | 1802,98 | -31 | 23 - 38 | 1 | 25,27 |
| | 1803,04 | 0 | 80 - 91 | 3 | 86, 88, 87 |
| 1931,13 | 1930,06 | -27 | 23 - 38 | 2 | 25 & 27 |
| 2058,19 | 2058,15 | -19 | 23 - 39 | 2 | 25 & 27 |
| 2149,18 | 2149,16 | -9 | 88 - 104 | 1 | 88, 99, 100 |
| 2376,04 | 2376,04 | 12 | 9 - 22 | 1 | 13 |
| 2494,34 | 2494,42 | 31 | 80 - 99 | 1 | 86, 87, 88 |
| 2622,37 | 2622,51 | 53 | 80 - 100 | 1 | 86, 87, 88, 99 |
| 2689,11 | 2689,28 | 62 | 6 - 22 | 1 | 7, 8, 13 |

(suite)

| $[M + H]^+$ Expérimental | Calculé | Déviation Séquence (ppm) | | Nombre de modification | Lysines modifiées |
|---|---|---|---|---|---|
| 3259,05 | 3259,54 | 150 | 1 - 22 | 1 | 5, 7, 8, 13 |
| 3299,04 | 3298,75 | -86 | 61 - 87 | 1 | 72, 73, 79, 86 |
| 3386,04 | 3386,61 | 169 | 1 - 22 | 2 | 5, 7, 8, 13 |
| 3426,05 | 3425,83 | -65 | 61 - 87 | 2 | 72, 73, 79, 86 |

Aucune ambiguïté sur la position des résidus aminoacides modifiés n'a été relevée lors de la prédiction (i. e. lorsque plusieurs sites de modification sont possibles sur un même peptide prédit).

Ce succès nous a conduit à réaliser des expériences de réticulation sur support solide avec des agents bifonctionnels.

**Exemple 2** : Agent homobifonctionnel sur support solide

2 - 1 Synthèse

Un agent de réticulation a été synthétisé sur différents supports solides en utilisant un bras espaceur de type adipoyl.

Les supports solides qui ont été utilisés sont : la résine PEGA, la résine ChemMatrix® et la résine macroporeuse Synbeads®.

Le diester NHS glutamate a été choisi comme agent de réticulation homobifonctionnel sur support solide. La voie de synthèse décrite ci-dessous (voir schéma 11) présentée sur le schéma 50 a été utilisée pour élaborer un agent de réticulation sur les différents supports solides : résine **36** (Synbeads), résine **37** (PEGA) et résine **38** (ChemMatrix).

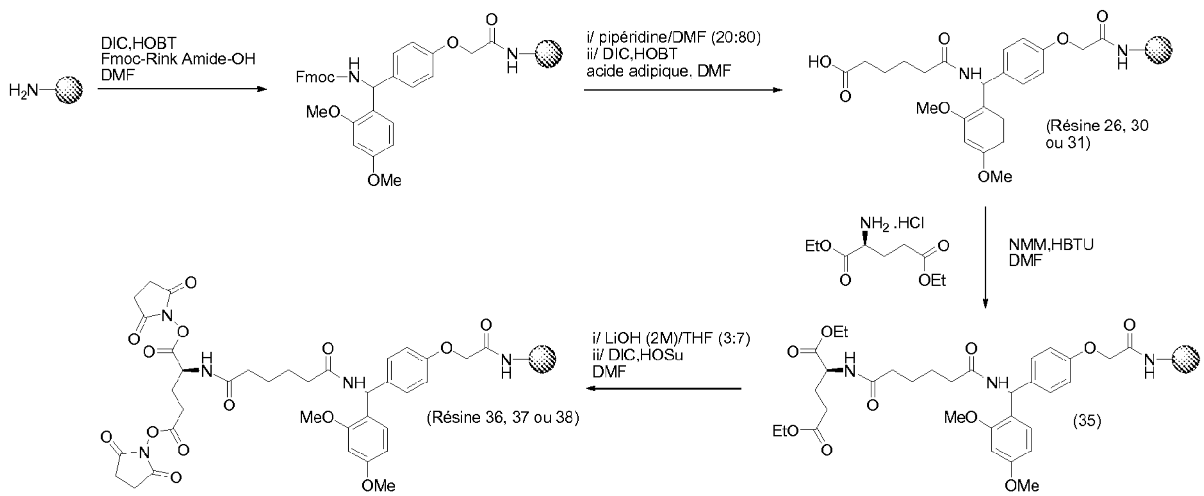


**Schéma 11 :** Synthèse d'un agent de réticulation homobifonctionnel sur support solide.

A titre d'exemple, seule la synthèse du bis(*N*-hydroxysuccinimdyl)-*N*-adipylamido-*L*-glutamate sur résine aminométhyl-méthacrylate Synbeads (0,81 mmol/g) est décrite.

○ *Fixation du linker Fmoc-Rink Amide sur la résine aminométhyl-méthacrylate Synbeads :*

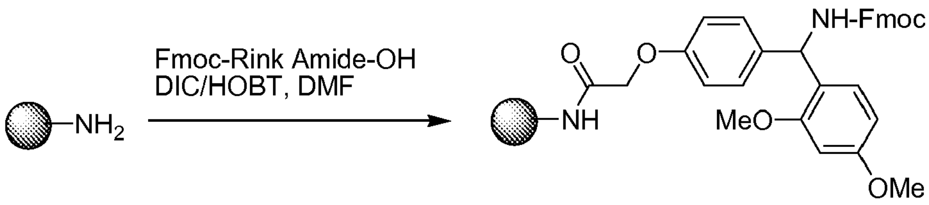


*Tableau récapitulatif des réactifs :*

| Réactifs | PM (g/mol) | d (mg/L) | Concentration Finale (mmol/L) | m ou v (mg ou µL) | Volume Solvant (mL) |
|---|---|---|---|---|---|
| Fmoc-Rink Amide-OH | 539,59 | 1 | 100 | 880 | 16,3 |
| DIC | 126,20 | 0,815 | 100 | 252 | 16,3 |
| HOBt | 135,13 | 1 | 100 | 220 | 16,3 |

*Protocole :*

0,815 g (0,66 mmol, 1 éq) de résine aminométhyl-méthacrylate Synbeads sont placés dans un réacteur en verre de 20 mL muni d'un fritté et conditionnés dans 10 mL de DCM pendant 5 minutes.
Après avoir été filtré, une solution de 16,3 mL DMF dans laquelle ont été mis successivement 880 mg (1,63 mmol, 2,5 éq) d'acide Fmoc-Rink Amide, 252 µL (1,62 mmol, 2,5 éq) de DIC et 220 mg (1,63 mmol, 2,5 éq) de HOBt, est placée dans le réacteur. Le milieu est agité mécaniquement pendant 2 heures.
La solution est retirée par filtration puis la résine est lavée successivement (2 fois 2 minutes pour chaque solvant) avec : DMF, méthanol et DCM, puis séchée sous vide.
Un test négatif au TNBS d'un échantillon montre l'absence de fonctions amines primaires.

○ *Déprotection du linker Fmoc-Rink Amide :*

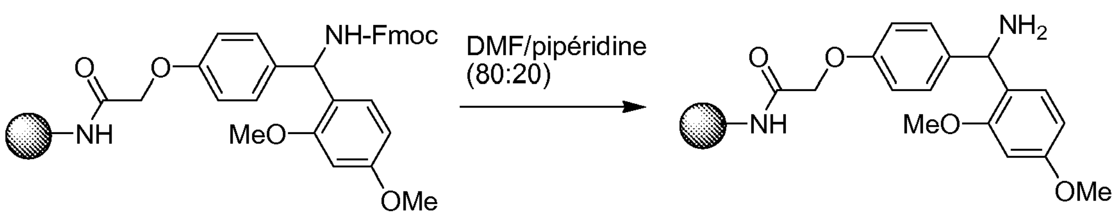


*Protocole :*

0,900 g de résine sont placés dans un réacteur en verre de 20 mL muni d'un fritté et conditionnés dans 10 mL de DCM pendant 5 minutes. Après avoir été filtré, la résine est mise en réaction avec une solution de DMF/pipéridine (80 : 20) : 2 fois 20 mL pendant 20 minutes.
La solution est retirée par filtration puis la résine est lavée successivement (2 fois 2 minutes pour chaque

solvant) avec : DMF, méthanol et DCM, puis séchée sous vide.

Un test positif au TNBS d'un échantillon montre la présence de fonctions amines primaires.

○ *Fixation de l'acide adipique :*

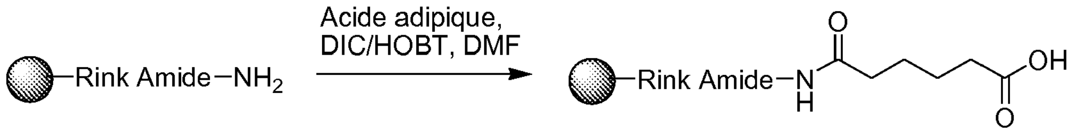


*Tableau récapitulatif des réactifs :*

| Réactifs | PM (g/mol) | d (mg/L) | Concentration Finale (mmol/L) | m ou v (mg ou μL) | Volume Solvant (mL) |
|---|---|---|---|---|---|
| Acide Adipique | 146,14 | 1 | 100 | 238 | 16,3 |
| DIC | 126,20 | 0,815 | 100 | 252 | 16,3 |
| HOBt | 135,13 | 1 | 100 | 220 | 16,3 |

*Protocole :*

Une solution de 16,3 mL DMF dans laquelle ont été mis successivement 238 mg (1,63 mmol, 2,4 éq) d'acide adipique, 252 μL (1,62 mmol, 2,4 éq) de DIC et 220 mg (1,63 mmol, 2,4 éq) de HOBt, est introduite dans un réacteur contenant 850 mg (0,69 mmol, 1 éq) de résine. Le milieu est placé sous agitation mécanique durant 2 heures.

Après filtration, la résine est alors lavée (2 fois 2 minutes pour chaque solvant) avec : DMF, méthanol et DCM, puis séchée sous vide.

Un test négatif au TNBS ainsi qu'un test positif au vert de Malachite de deux échantillons de résine montrent l'absence de fonctions amine primaire et la présence de fonctions acide carboxylique sur la résine.

○ *Fixation du L-glutamate de diéthyle :*

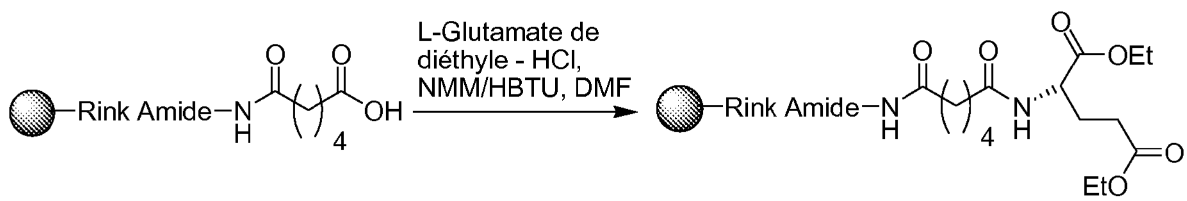


*Tableau récapitulatif des réactifs :*

| Réactifs | PM (g/mol) | d (mg/L) | Concentration Finale (mmol/L) | m ou v (mg ou μL) | Volume Solvant (mL) |
|---|---|---|---|---|---|
| L-Glutamate diéthyle - HCl | 239,70 | 1 | 100 | 391 | 16,3 |
| NMM | 101,15 | 0,92 | 200 | 358 | 16,3 |

(suite)

| Réactifs | PM (g/mol) | d (mg/L) | Concentration Finale (mmol/L) | m ou v (mg ou µL) | Volume Solvant (mL) |
|---|---|---|---|---|---|
| HBTU | 379,25 | 1 | 100 | 618 | 16,3 |

*Protocole :*

Une solution de 16,3 mL DMF contenant 391 mg (1,63 mmol, 2,1 éq) de chlorhydrate de L-Glutamate de diéthyle et 358 µL de NMM (3,26 mmol, 4,2 éq) est introduite dans un réacteur contenant 950 mg (0,77 mmol, 1 éq) de résine. Après 2 minutes d'agitation, 628 mg (1,63 mmol, 2,1 éq) de HBTU sont introduits dans le milieu. La résine est laissée sous agitation mécanique pendant 2 heures.

Après filtration, la résine est lavée successivement (2 fois 2 minutes pour chaque solvant) avec : DMF, méthanol et DCM, puis séchée sous vide.

Un test au vert de Malachite négatif de Malachite montre l'absence de fonctions acide carboxylique sur la résine.

○ *Saponification des esters carboxyliques :*

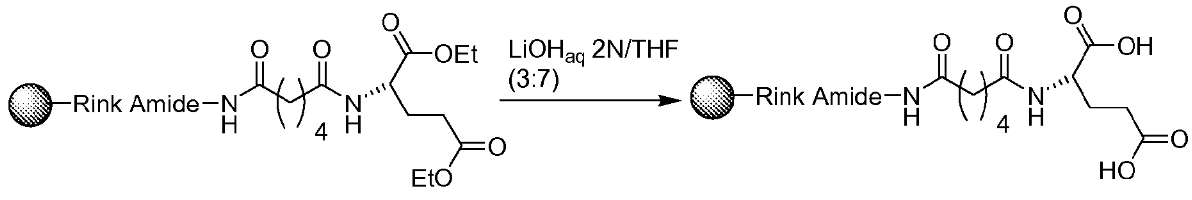


*Protocole :*

20 mL d'un mélange $LiOH_{aq}$ 2N/THF (3 : 7) sont placés dans un réacteur contenant 1 g de résine. Le milieu est agité mécaniquement pendant 4 heures.

Après filtration du milieu, la résine est lavée successivement (2 fois 2 minutes pour chaque solvant) avec : eau distillée, méthanol, DMF, méthanol et DCM.

Un test positif au vert de Malachite montre la présence de fonctions acide carboxylique sur la résine.

○ *Activation en ester N-hydroxysuccinimide :*

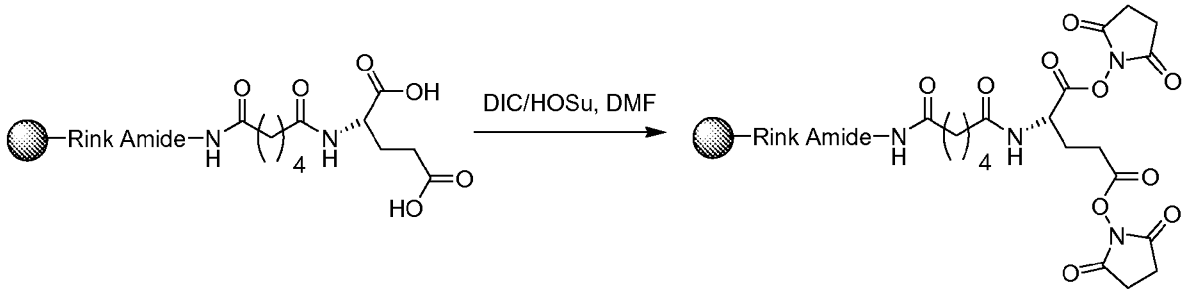

*Tableau récapitulatif des réactifs :*

| Réactifs | PM (g/mol) | d (mg/L) | Concentration Finale (mmol/L) | m ou v (mg ou μL) | Volume Solvant (mL) |
|---|---|---|---|---|---|
| DIC | 126,20 | 0,815 | 100 | 31 | 2 |
| HOSu | 115,09 | 1 | 100 | 23 | 2 |

*Protocole :*

100 mg de résine (0,081 mmol, 1 éq) sont conditionnés avec 3 mL de DCM dans une seringue munie d'un fritté pendant 3 minutes.

Après filtration du solvant, une solution de 2 mL de DMF contenant 31 μL (0,2 mmol, 2,5 éq) de DIC est placée dans la seringue. La résine est agitée pendant 5 minutes. 23 mg (0,2 mmol, 2,5 éq) de HOSu sont ajoutés. Le milieu est laissé sous agitation pendant 1 heure.

Après filtration du milieu, la résine est lavée successivement (2 fois 2 minutes pour chaque solvant) avec : DMF et DCM puis séchée sous vide.

2 - 2 - Test de réticulation d'un peptide modèle

Les trois résines **36, 37** et **38** ont été utilisées pour fixer le peptide modèle **34.** Les expériences ont été menées selon le protocole utilisé pour la fixation de peptide sur des agents monofonctionnels supportés (Exemple 1).

Seule la résine **36** utilisant le support solide Synbeads a permis d'immobiliser suffisamment de peptide **34** pour obtenir un spectre de masse.

Ce spectre montre que l'étape de lavage de la résine n'a pas été totalement efficace car on observe un ion de valeur m/z 818,3 Da correspondant au peptide **34** non modifié. En revanche, plusieurs ions détectés (m/z : 945,5 Da, 1201,1 Da, 1330,8 Da) correspondent à des modifications du peptide **34** avec un ou deux bras monofonctionnels adipoyl. Par conséquent, la réaction de couplage du glutamate de diéthyle sur le bras monofonctionnel n'a pas été quantitative et l'étape d'activation finale a aussi activé l'acide adipique ce qui montre que le couplage permettant d'obtenir le diester **35** n'est pas quantitatif.

L'analyse montre aussi que l'ion dont la masse est de 1074,6 Da est le plus intense. Celui-ci correspond à une modification de type 0 du peptide **34.**

On observe également un ion à 1874,2 Da correspondant à la réticulation de type 2 de deux peptides **34.** Ceci est particulièrement intéressant car les réticulations de type 2 permettent de fournir des données importantes lors de l'étude structurale de protéines.

2 - 3 - Réticulation du cytochrome c sur support solide

Les trois résines **36, 37** et **38** ont été utilisées afin d'effectuer des expériences d'immobilisation du cytochrome c sur support solide. Ces expériences ont été menées dans les mêmes conditions que pour les agents monofonctionnels supportés.

Seule la résine **36** utilisant le support solide Synbeads a permis d'immobiliser suffisamment de protéine pour obtenir un spectre de masse.

**<u>Exemple 3 : Analyses par spectrométrie de masse MALDI - TOF</u>**

Le spectromètre de masse qui a été utilisé est de la gamme Bruker : Ultraflex Daltonics. Les logiciels d'acquisition de spectre et d'exploitation de données sont respectivement : Flexcontrol et Flexanalysis version 2.2.

Le spectromètre est équipé d'une source SCOUT et la désorption/ionisation est effectuée à l'aide d'un laser à azote de longueur d'onde 337 nm et la fréquence utilisée est de 50 Hz. La puissance du laser est modulable grâce à un atténuateur.

3 - 1 - L'étalonnage

L'étalonnage des analyses par spectrométrie MALDI - TOF a été effectuée en mode externe. Deux kits d'étalonnage ont été utilisés :

- « Peptide calibration standard II » (Bruker #222570) : allant de 700 à 3200 Th

- « Protein calibration standard I » (Bruker #206355) : allant de 5000 à 17500 Th

L'étalonnage externe est effectué en faisant un dépôt de l'un de ces mélanges commerciaux avec une matrice appropriée (*e.g.* acide $\alpha$-cyano-4-hydroxycinnamique (HCCA) pour le « Peptide calibration standard II », acide sinapinique (AS) pour le « Protein calibration standard I ») à proximité du ou des dépôt(s) à analyser.

3 - 2 - Préparation de l'échantillon

Les échantillons sont déposés sur une plaque Bruker Daltonics MTP 384 cibles en acier poli, suivant deux méthodes.

❖ **Le dépôt en goutte séchée**

Le dépôt goutte à goutte a été préféré dans la plupart des analyses utilisant les matrices « classiques » HCCA et AS. Il est réalisé par co-cristallisation d'une goutte de solution matricielle et d'une goutte d'une solution d'analyte. Chaque dépôt peut contenir entre 0,5 et 1 $\mu$L de chaque solution.

Les solutions de matrice HCCA et AS sont préparées par saturation dans une solution eau/ACN/TFA (50 : 50 : 0,1).

- Analyses avec la matrice acide sinapinique de tests de modifications sur protéines avant digestion :

0,5 $\mu$L (0,5 pmol) de solution protéique à 1 $\mu$M dans un mélange eau/ACN/TFA (50 : 50 : 0,1) sont co-cristallisés avec 0,5 $\mu$L d'acide sinapinique saturé dans un mélange eau/ACN/TFA (50 : 50 : 0,1).

- Analyses avec la matrice acide HCCA de tests de modifications sur protéines après digestion enzymatique :

0,5 $\mu$L (0,5 pmol) de solution peptidique à 1 $\mu$M dans un mélange eau/ACN/TFA (50 : 50 : 0,1) sont co-cristallisés avec 0,5 $\mu$L d'acide HCCA saturé dans un mélange eau/ACN/TFA (50 : 50 : 0,1).

**<u>Liste de références</u>**

Atherton, E.; Clive, D. L. J.; Sheppard, R. C. Journal of the American Chemical Society 1975, 97, 6584-6585.
Meldal, M. Tetrahedron Letters 1992, 33, 3077-3080.

**Revendications**

1. Agent de réticulation des protéines sur support solide de formule (I)

M-L-B-A (I)

dans laquelle
M est un support solide choisi dans le groupe constitué des polymères naturels et dérivés, des matrices inorganiques et des polymères de synthèse fonctionnalisés,
L est un linker,
la liaison entre L et B est clivable,
B est une chaîne linéaire hydrocarbonée en $C_1$ à $C_{60}$, de préférence $C_5$ à $C_{10}$, présentant éventuellement une ou plusieurs double ou triple liaisons dont un ou plusieurs carbones peuvent être remplacés par O, NH, -C=O,

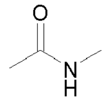

ou

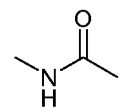

et un ou plusieurs autres carbones peuvent être substitués par un groupe $C_{1-10}$ alkyle, $C_{2-10}$ alcényle, $C_{2-10}$ alcynyle, aryle ou aryl($C_{1-10}$ alkyle), et
B forme avec L et A une liaison amide, et
A est un agent de réticulation des protéines présentant la formule (II) ou (IIbis) ou (III) ou (IIIbis)

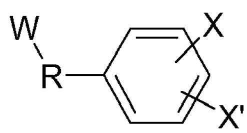 (II) 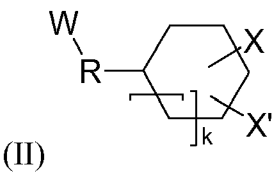 (IIbis)

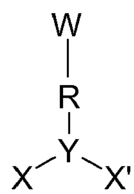 (III)

-W-R-X (IIIbis)

dans lesquelles
W est NH ou C=O,
Y est N ou CH,
R est absent ou représente un groupe $C_{1-10}$ alkyle, $C_{2-10}$ alcényle ou $C_{2-10}$ alcynyle,
k est 0, 1, 2 ou 3,
X et X', identiques ou différents, sont une fonction réactive des protéines.

**2.** Agent de réticulation selon la revendication 1, où B présente la formule (IV)

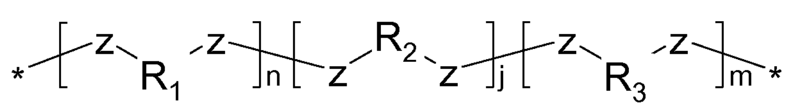

(IV)

dans laquelle Z est NH ou C=O,
n et m sont 1 ou 0,
j est un entier compris entre 1 et 60.
$R_1$, $R_2$ et $R_3$, identiques ou différents, sont une chaîne hydrocarbonée en $C_1$ à $C_6$ dont un ou plusieurs carbones peuvent être remplacés par O et un ou plusieurs autres carbones peuvent être substitués par un groupe alkyle, aryle ou arylalkyle.

**3.** Agent de réticulation selon la revendication 1 ou 2, où B présente l'une des formules (V) à (IX)

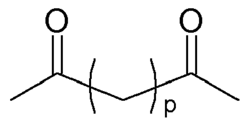

(V)

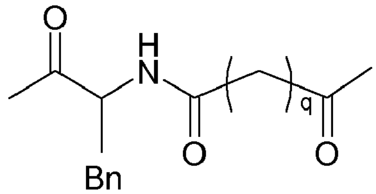

(VI)

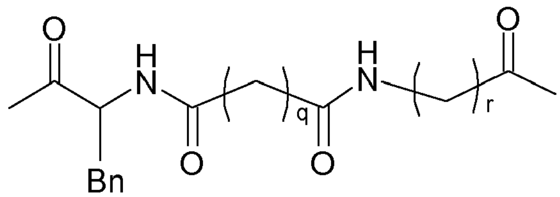

(VII)

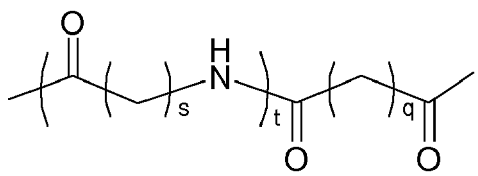

(VIII)

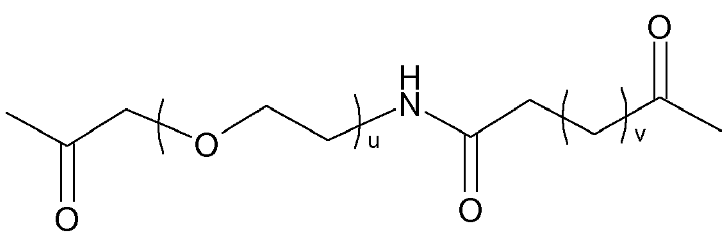

(IX)

dans lesquelles p, q, r, s, u et v sont des entiers de 1 à 5 et t est 1 ou 0.

4. Agent de réticulation selon l'une quelconque des revendications précédentes, où M est une matrice macroporeuse de type méthacrylique.

5. Agent de réticulation selon l'une quelconque des revendications précédentes, où L est un linker acido-labile.

6. Agent de réticulation selon la revendication 5, où L est un linker Rink-Amide.

7. Agent de réticulation selon l'une quelconque des revendications précédentes, où X et X', identiques ou différents, sont choisis dans le groupe constitué des fonctions imidoester, ester *N*-hydroxysuccinimide, isocyanate, isothiocyanate, *N*-maléimide, disulfure, 1,2-dicarbonyle, benzophénone et arylazide.

**8.** Procédé de préparation d'un agent de réticulation selon l'une quelconque des revendications 2 à 7, comprenant les étapes suivantes :

(i)

- éventuellement couplage peptidique entre la fonction amine du linker et la fonction acide carboxylique d'un acide aminé dont la fonction amine est protégée, puis déprotection de la fonction amine,
- éventuellement couplage par réaction d'estérification entre la fonction alcool du linker et la fonction acide carboxylique d'un acide aminé dont la fonction amine est protégée, puis déprotection de la fonction amine.
- éventuellement couplage par réaction d'estérification entre la fonction halogène du linker et la fonction acide carboxylique sous forme de carboxylate d'un acide aminé dont la fonction amine est protégée, puis déprotection de la fonction amine.
- éventuellement couplage peptidique entre la fonction acide du linker et la fonction amine d'une diamine dont une des fonctions amines peut être protégée, puis déprotection de la fonction amine,
- éventuellement couplage par formation d'ester entre la fonction acide du linker et la fonction alcool d'un amino alcool dont la fonction amine est protégée, puis déprotection de la fonction amine,
- éventuellement couplage peptidique entre la fonction amine du linker et un diacide ou un anhydride et
- éventuellement couplage peptidique entre la fonction amine du linker et un diacide dont une fonction est protégée sous forme d'ester et puis déprotection de la fonction acide protégée.
- éventuellement couplage par estérification entre la fonction alcool du linker et un diacide ou un anhydride et,
- éventuellement couplage par estérification entre la fonction alcool du linker et un diacide dont une fonction est protégée sous forme d'ester et puis déprotection de la fonction acide protégée.
- éventuellement couplage par réaction d'estérification entre la fonction halogène du linker et la fonction acide carboxylique sous forme de carboxylate d'un diacide dont une fonction est protégée sous forme d'ester puis déprotection de la fonction acide protégée.
- éventuellement couplage par réaction d'estérification entre la fonction halogène du linker et la fonction acide carboxylique sous forme de carboxylate d'un diacide

(ii) répétition une ou plusieurs fois des étapes suivantes afin d'obtenir le bras espaceur désiré

- couplage peptidique entre la fonction acide du composé obtenu à l'étape précédente et la fonction amine d'un acide aminé dont la fonction acide est protégée, puis déprotection de la fonction acide
- couplage peptidique entre la fonction amine du composé obtenu à l'étape précédente et la fonction acide carboxylique d'un acide aminé dont la fonction amine est protégée, puis déprotection de la fonction amine,
- couplage par réaction d'estérification entre la fonction alcool du composé obtenu à l'étape précédente et la fonction acide carboxylique d'un acide aminé dont la fonction amine est protégée, puis déprotection de la fonction amine.
- couplage peptidique entre la fonction acide du composé obtenu à l'étape précédente et la fonction amine d'une diamine dont une des fonctions amines peut être protégée, puis déprotection de la fonction amine,
- couplage par formation d'ester entre la fonction acide du composé obtenu à l'étape précédente et la fonction alcool d'un amino alcool dont la fonction amine est protégée, puis déprotection de la fonction amine,
- couplage peptidique entre la fonction amine du composé obtenu à l'étape précédente et un diacide ou un anhydride et
- couplage peptidique entre la fonction amine du composé obtenu à l'étape précédente et un diacide dont une fonction est protégée sous forme d'ester et puis déprotection de la fonction acide protégée.
- couplage par estérification entre la fonction alcool du composé obtenu à l'étape précédente et un diacide ou un anhydride et,
- couplage par estérification entre la fonction alcool du composé obtenu à l'étape précédente et un diacide dont une fonction est protégée sous forme d'ester et puis déprotection de la fonction acide protégée.

**9.** Utilisation d'un agent de réticulation sur support solide selon l'une quelconque des revendications 1 à 8 pour isoler les peptides réticulés avant leur analyse en spectrométrie de masse.

**10.** Méthode d'analyse structurale d'une protéine ou d'un complexe de protéines comprenant les étapes suivantes :

a) réticulation de la protéine ou du complexe protéique sur l'agent de réticulation sur support solide selon l'une quelconque des revendications 1 à 8 par les fonctions X et/ou X',
b) lavage(s) pour éliminer les protéines ou complexes de protéines non réticulés,

c) digestion enzymatique de la protéine ou complexe de protéines fixé à l'agent de réticulation sur support solide selon l'une quelconque des revendications 1 à 8,
d) lavage(s) pour éliminer les peptides non réticulés,
e) clivage du support solide entre M et B, et
f) analyse par spectrométrie de masse.

**11.** Méthode selon la revendication 11, les lavages étant réalisés avec un ou plusieurs solvants aqueux ou organiques.

**12.** Méthode selon la revendication 12, les lavages étant réalisés avec un ou plusieurs solvants choisis dans le groupe constitué de tampon PBS, solution aqueuse de HCl, solution aqueuse de NaOH, eau, méthanol, DMF, DCM.

**13.** Méthode selon l'une quelconque des revendications 11 à 13, la digestion enzymatique étant réalisée par la trypsine.

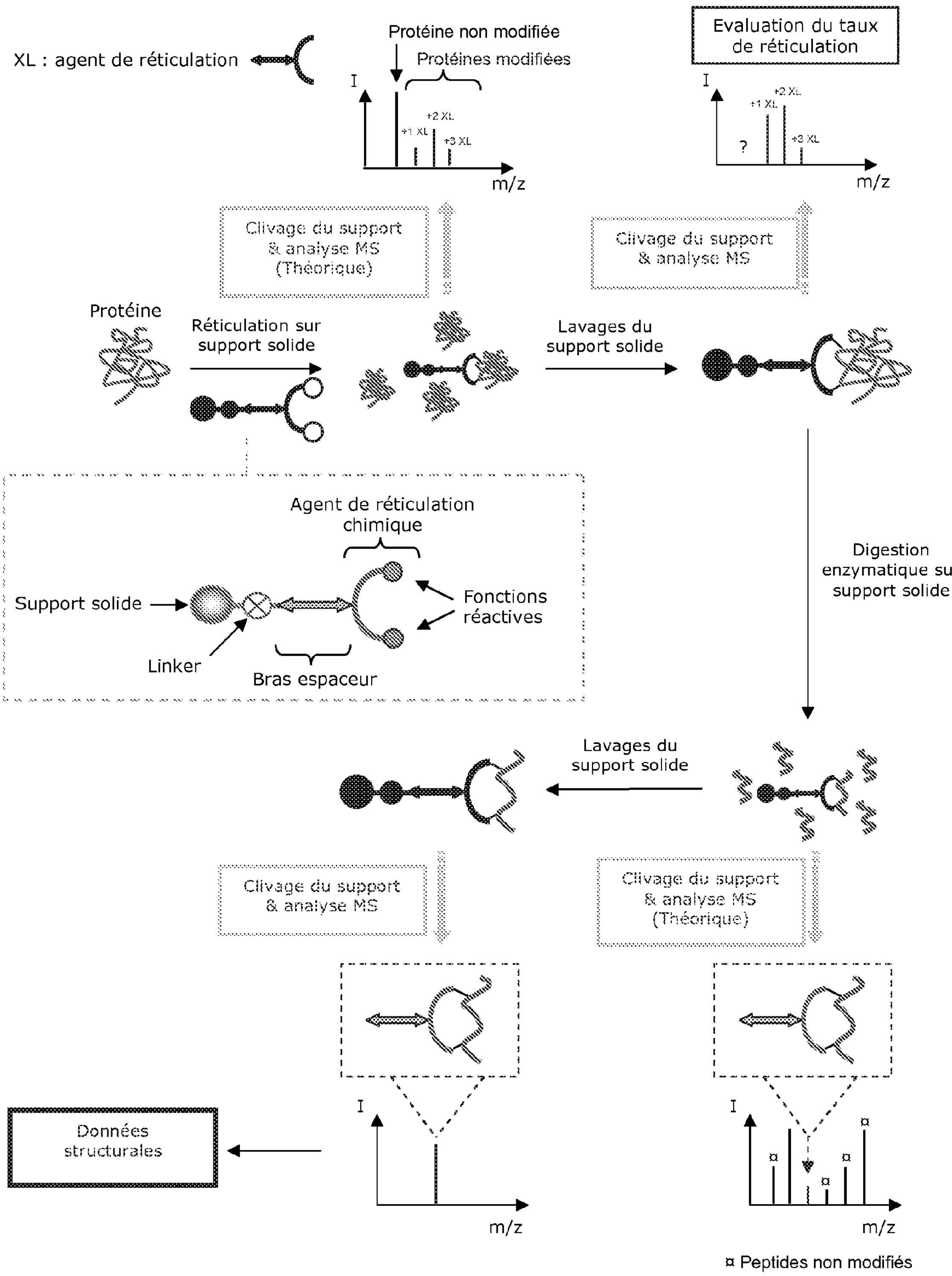

XL : agent de réticulation
Protéine non modifiée
Protéines modifiées
I
+1 XL
+2 XL
+3 XL
m/z
Evaluation du taux de réticulation
?
Clivage du support & analyse MS (Théorique)
Clivage du support & analyse MS
Protéine
Réticulation sur support solide
Lavages du support solide
Agent de réticulation chimique
Support solide
Fonctions réactives
Linker
Bras espaceur
Digestion enzymatique sur support solide
Données structurales
¤ Peptides non modifiés


Fig.1

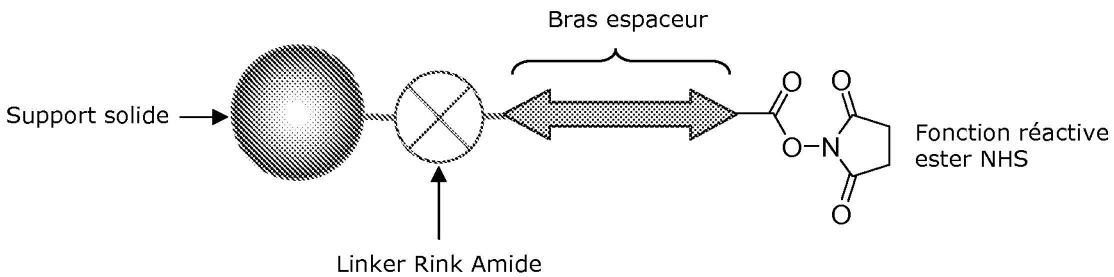
Bras espaceur
Support solide
Fonction réactive
ester NHS
Linker Rink Amide

Fig. 2

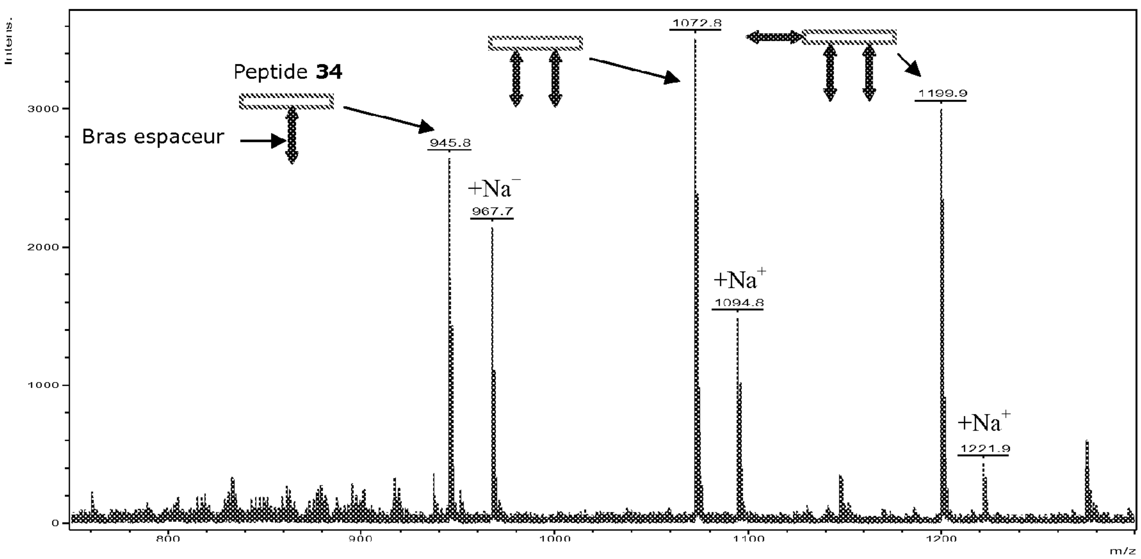
Intens.
Peptide 34
Bras espaceur
945.8
+Na+
967.7
1072.8
+Na+
1094.8
1199.9
+Na+
1221.9
m/z

**Fig. 3**

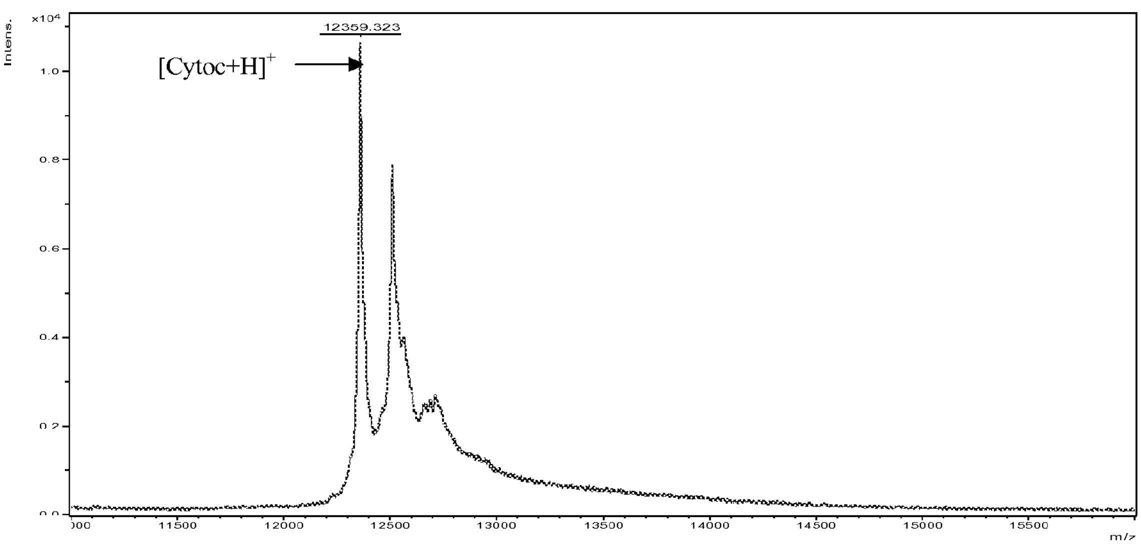
x10⁴
Intens.
12359.323
[Cytoc+H]+
m/z

**Fig. 4**

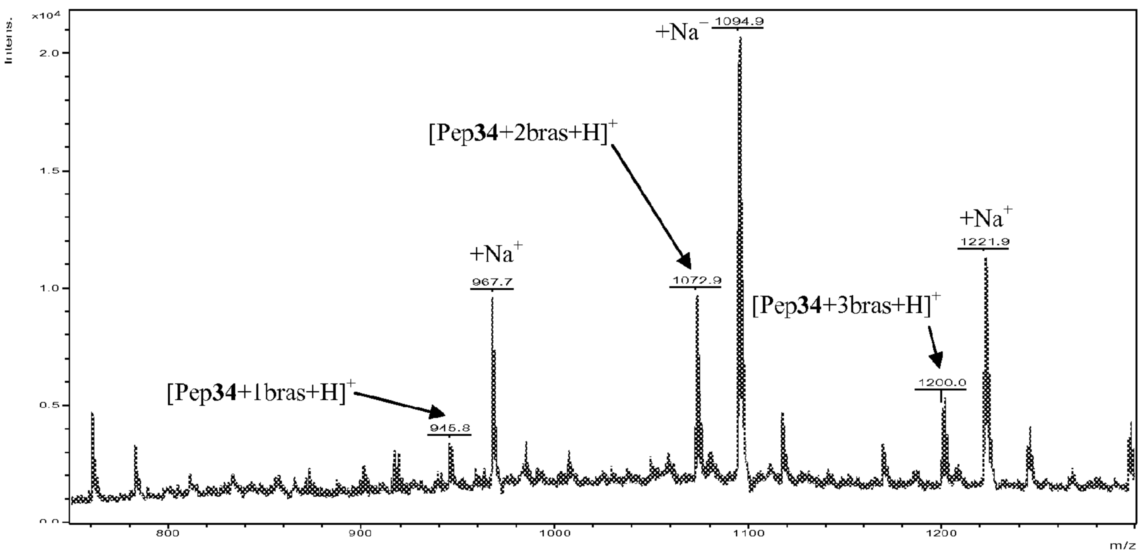
Intens.
x10⁴
+Na⁻
1094.9
[Pep34+2bras+H]⁺
+Na⁺
1221.9
+Na⁺
967.7
1072.9
[Pep34+3bras+H]⁺
1200.0
[Pep34+1bras+H]⁺
945.8
m/z

**Fig. 5**

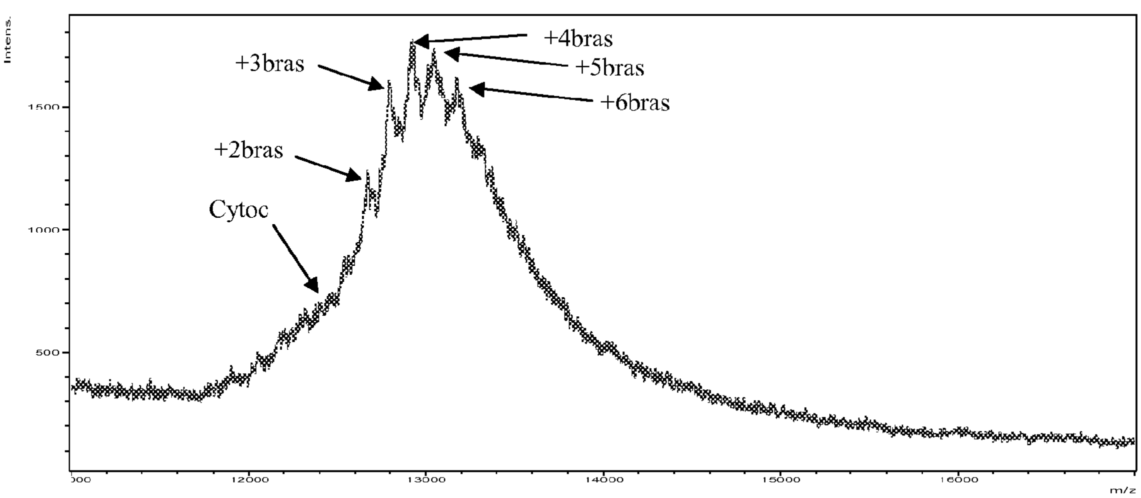
Intens.
+4bras
+3bras
+5bras
+6bras
+2bras
Cytoc
m/z

**Fig. 6**

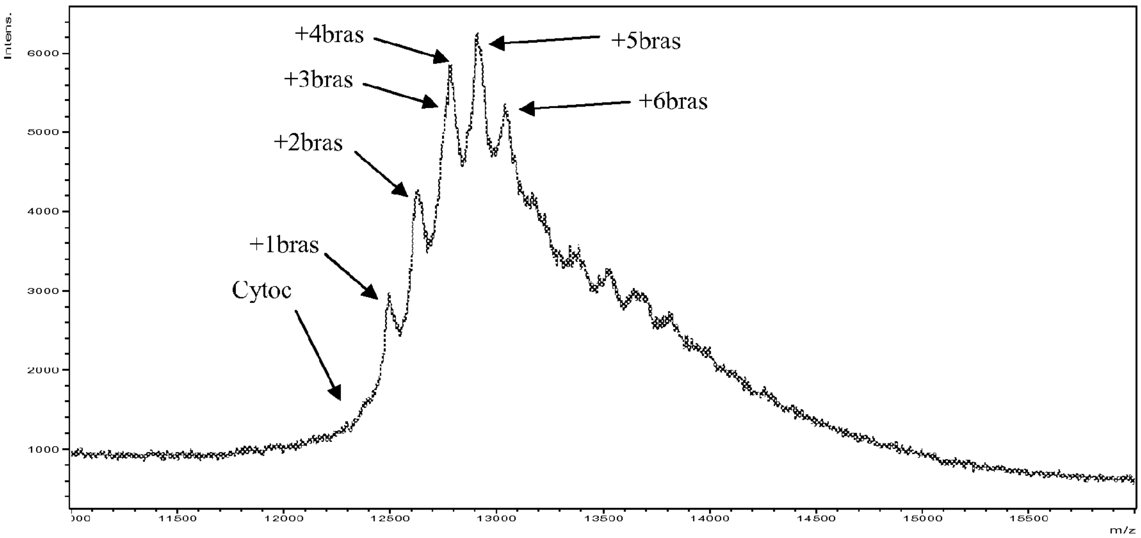
Intens.
6000
5000
4000
3000
2000
1000
+4bras
+5bras
+3bras
+6bras
+2bras
+1bras
Cytoc
11500
12000
12500
13000
13500
14000
14500
15000
15500
m/z

**Fig. 7**

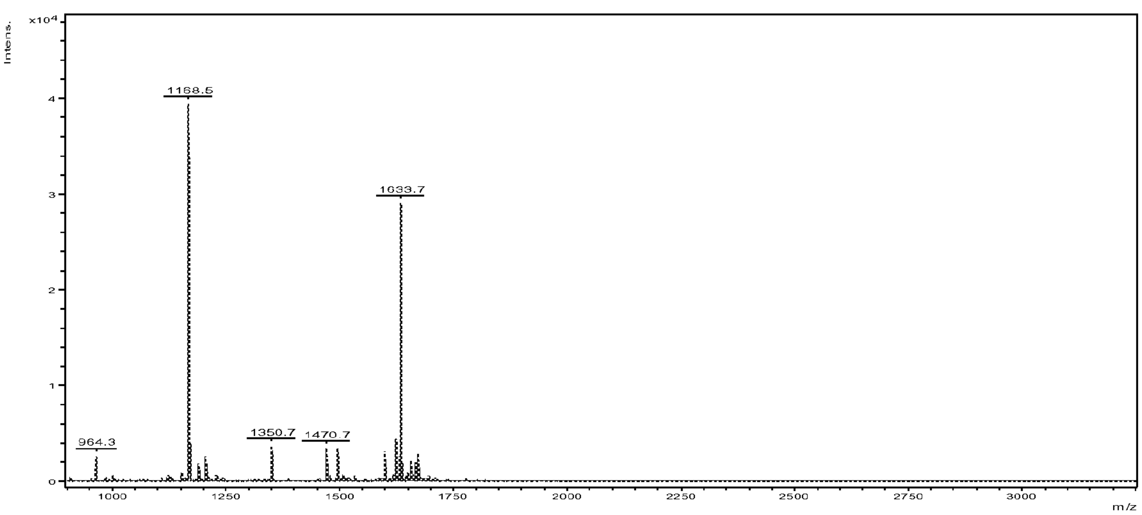
Intens.
x10⁴
4
3
2
1
0
1168.5
1633.7
964.3
1350.7
1470.7
1000
1250
1500
1750
2000
2250
2500
2750
3000
m/z

**Fig. 8**

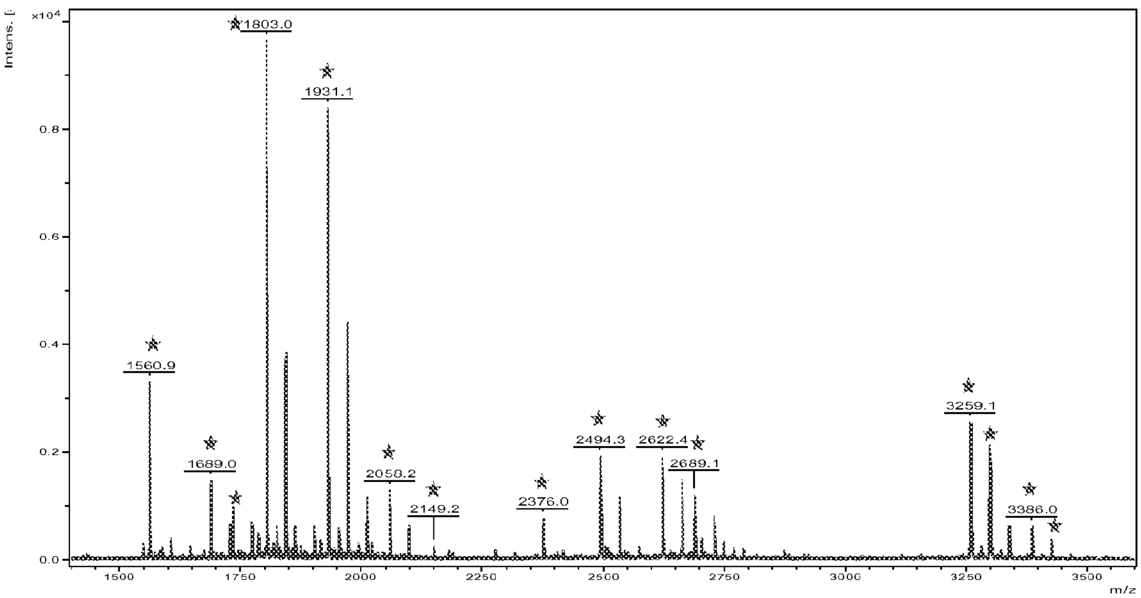
Intens.
x10⁴
0.8
0.6
0.4
0.2
0.0
1803.0
1931.1
1560.9
1689.0
2058.2
2149.2
2376.0
2494.3
2622.4
2689.1
3259.1
3386.0
1500
1750
2000
2250
2500
2750
3000
3250
3500
m/z

Fig. 9

Europäisches Patentamt
European Patent Office
Office européen des brevets

## RAPPORT DE RECHERCHE EUROPEENNE

Numéro de la demande
EP 09 30 5200

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (IPC) |
| X | KIYONAKA S ET AL.: "Combinatorial library of low molecular-weight organo- and hydrogelators based on glycosylated amino acid derivatives by solid-phase synthesis" CHEMISTRY - A EUROPEAN JOURNAL, vol. 9, no. 4, 17 février 2003 (2003-02-17), pages 976-983, XP9119290 WILEY-VCH VERLAG (11-02-2003: on line) DOI: 10.1002/chem.200390120 * figure 1 * ----- | 1-3,5,8 | INV. G01N33/68 |
| X | KIM SOYEON ET AL.: "Synthetic MMP-13 degradable ECMs based on poly(N-isopropylacrylamide- co-acrylic acid) semi-interpenetrating polymer networks. I. Degradation and cell migration." JOURNAL OF BIOMEDICAL MATERIALS RESEARCH. PART A, vol. 75, no. 1, 1 octobre 2005 (2005-10-01), pages 73-88, XP9119211 (27-07-2005: on line) ISSN: 1549-3296 DOI: 10.1002/jbm.a.30375 * Scheme 1; abrégé * ----- | 1-2,4, 7-8 | DOMAINES TECHNIQUES RECHERCHES (IPC) G01N |
| X | PARSONS J G ET AL.: "A Review of Solid-Phase Organic Synthesis on SynPhase(TM) Lanterns and SynPhase(TM) Crowns" METHODS IN ENZYMOLOGY, vol. 369, 2003, pages 39-74, XP9119227 US (27-02-2004: on line) ISSN: 0076-6879 DOI: 10.1016/S0076-6879(03)69003-8 * Schemes 2, 10, 17, 18, 26 * * figure 3 * ----- -/-- | 1-2,4-8 | |

Le présent rapport a été établi pour toutes les revendications

1

| Lieu de la recherche | Date d'achèvement de la recherche | Examinateur |
|---|---|---|
| Munich | 6 août 2009 | Jenn, Thierry |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Europäisches Patentamt
European Patent Office
Office européen des brevets

## RAPPORT DE RECHERCHE EUROPEENNE

Numéro de la demande
EP 09 30 5200

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2003/068379 A1 (LI FRANK Q [US] ET AL) 10 avril 2003 (2003-04-10)<br>* revendications 20,45; figures 1,6,13 * | 1,5 | |
| X | US 2005/261475 A1 (TSENG HUANG-CHUN [US] ET AL) 24 novembre 2005 (2005-11-24)<br>* revendications 12-16,19-39; figure 1 * | 1-2,5-8 | |
| X | CHEN Y-J ET AL.: "Carbohydrate-encapsulated gold nanoparticles for rapid target-protein identification and binding-epitope mapping" CHEMBIOCHEM - A EUROPEAN JOURNAL OF CHEMICAL BIOLOGY, vol. 6, no. 7, 25 mai 2005 (2005-05-25), pages 1169-1173, XP002498396 WILEY VCH, WEINHEIM, DE (24-05-2005: on line) ISSN: 1439-4227 DOI: 10.1002/cbic.200500023 | 1,8-9 | |
| Y | * Schemes 1 and 2 * | 10-13 | DOMAINES TECHNIQUES RECHERCHES (IPC) |
| Y | EP 1 296 143 A2 (SEQUENOM INC [US]) 26 mars 2003 (2003-03-26)<br>* revendications 1,10,11 *<br>* page 38, alinéa 251 *<br>* page 4, alinéa 17 *<br>* page 4, alinéa 20 *<br>* page 6, alinéa 38 - alinéa 39; figures 2,3 *<br>* page 24, alinéa 168 *<br>* page 22, alinéa 155 *<br>* page 39, alinéa 254 - alinéa 255 * | 10-13 | |

1

Le présent rapport a été établi pour toutes les revendications

| Lieu de la recherche | Date d'achèvement de la recherche | Examinateur |
|---|---|---|
| Munich | 6 août 2009 | Jenn, Thierry |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE**
**RELATIF A LA DEMANDE DE BREVET EUROPEEN NO.** EP 09 30 5200

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

06-08-2009

| Document brevet cité au rapport de recherche | | Date de publication | Membre(s) de la famille de brevet(s) | Date de publication |
|---|---|---|---|---|
| US 2003068379 | A1 | 10-04-2003 | AUCUN | |
| US 2005261475 | A1 | 24-11-2005 | AUCUN | |
| EP 1296143 | A2 | 26-03-2003 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 08305512 A
- FR 1295537

### Littérature non-brevet citée dans la description


- **Atherton, E. ; Clive, D. L. J. ; Sheppard, R. C.** *Journal of the American Chemical Society,* 1975, vol. 97, 6584-6585
- **Meldal, M.** *Tetrahedron Letters,* 1992, vol. 33, 3077-3080